# EUROPÄISCHE PATENTSCHRIFT

(11) **EP 1 793 885 B1**
(45) Veröffentlichungstag und Bekanntmachung des Hinweises auf die Patenterteilung: **28.09.2016**
(21) Anmeldenummer: 05771006.3
(22) Anmeldetag: 22.07.2005
(51) Int. Cl.: A61M 16/06, A62B 9/00, A61L 29/14, A61L 27/00

(54) **KUNSTSTOFFE FÜR MEDIZINTECHNISCHE GERÄTE**
PLASTICS FOR MEDICAL TECHNICAL DEVICES
MATIERES PLASTIQUES POUR APPAREILS MEDICAUX

(30) Priorität: 03.09.2004 DE 102004043208; 18.03.2005 DE 102005013079; 14.06.2005 DE 102005027724
(43) Veröffentlichungstag der Anmeldung: 13.06.2007
(60) Teilanmeldung: 12007111.3
(73) Patentinhaber: Löwenstein Medical Technology GmbH + Co. KG, 22525 Hamburg (DE)
(72) Erfinder: FELDHAHN, Karl-Andreas, 22761 Hamburg (DE); SCHULZ, Gerd, 22869 Schenefeld (DE); EIFLER, Martin, 25348 Glückstadt (DE); FRERICHS, Arnold, 21614 Buxtehude (DE); MARX, Thomas, 20144 Hamburg (DE); VITT, Elmar, 27356 Rotenburg (DE)
(74) Vertreter: Patentanwälte Klickow & Partner Partnerschaftsgesellschaft mbB
(86) Internationale Anmeldenummer: PCT/DE2005/001289
(87) Internationale Veröffentlichungsnummer: WO 2006/024253

(56) Entgegenhaltungen:
- EP-A1- 1 318 228
- EP-A2- 0 933 388
- EP-A2- 1 040 874
- WO-A1-02/49980
- WO-A2-03/076169
- US-A- 4 677 143
- US-A1- 2003 147 932
- US-A1- 2003 221 691
- US-A1- 2004 079 374

## Beschreibung

Die Erfindung betrifft ein Beatmungsgerät bestehend aus einer Luftfördereinrichtung, einem Atemgasschlauch und einer Beatmungsmaske, wobei die Luftfördereinrichtung über den Atemgasschlauch an die Beatmungsmaske angeschlossen ist, wobei die Beatmungsmaske als ein modulartiges System ausgebildet ist und ein Bauteil ausgebildet als ein Formkörper aus Kunststoff aufweist, wobei mindestens ein Bereich des Formkörpers eine Oberfläche aufweist, die keimabweisende und/oder biozide und/oder antimikrobielle Eigenschaften aufweist. Ferner betrifft die Erfindung ein Verfahren ein Verfahren zur Herstellung eines erfindungsgemäßen definierten Formkörpers.

Medizintechnische Geräte können eine bakterielle Belastung beim Nutzer verursachen. Möglichen Quellen sind: die Produktion, der Service, die Klinik und der Patient/Nutzer.

Bei immunkompetenten Nutzern ist die Infektionsgefahr vom Gerät ausgehend relativ gering. Bei immunsuprimierten Nutzern stellt sich die Situation wesentlich problematischer dar. Daher sind geeignete Maßnahmen zumindest für den Patientenwechsel am Gerät zu ergreifen, um eine mögliche Keimbelastung zu reduzieren / zu entfernen. Der wesentliche An-EP 05 771 006.3 teil an Keimen sammelt sich im Inneren der Geräte. Dies ist insbesondere bei CPAP-, Beatmungs-, Absaug- und anderen medizintechnischen Geräten problematisch.

Die mechanische Desinfektion von CPAP-, Beatmungs-, Absaug- und anderen medizintechnischen Geräten ist bei der Verwendung der zurzeit üblichen Werkstoffe als problematisch, ineffizient und unwirtschaftlich zu bezeichnen. Das Zerlegen der Geräte, Einsprühen mit Flächendesinfektionsmitteln, Einlegen der Einzelteile in Desinfektionsmittel und anschließende Neumontage der Geräte ist mit einem hohen Aufwand verbunden. Dazu kommt ein nur geringer Erfolg dieser Maßnahmen. Alle bisher nachgewiesenen Keime halten sich in den porösen Oberflächen der verwendeten Materialien und widerstehen oft den mechanischen Desinfektionsversuchen. Nach dem Stand der Technik werden medizintechnische Geräte daher ordnungsgemäß einer Desinfektion unterzogen und/oder autoklaviert.

Eine erfolgreiche Desinfektion kann mit einer Formaldehyd - Begasung im Aseptor erreicht werden. Bei diesem Verfahren werden die Geräte in einer abgeschlossenen Edelstahlkammer bei einer Temperatur von 45 - 50° C, einer Luftfeuchtigkeit von mindestens 85% und einer Formaldehydkonzentration von ca. 3000 ppm begast und anschließend ausreichend nachgelüftet. Aus Sicherheitsgründen ist die Mindestlüftungszeit auf 220 Stunden festgelegt worden.

Die Voraussetzung für diese Art der Desinfektion ist die Verwendung von aldehydbeständigen Werkstoffen. Wesentlich ist auch eine gerätebezogene technische und anwendungstechnische Schulung des Personals in der Desinfektionsanlage, um auf die gerätespezifischen Besonderheiten der unterschiedlichen Geräte eingehen zu können. Diese Maßnahmen sind teuer und zeitaufwendig.

Aus der US 2004/0079374 A1 ist bereits ein Beatmungsgerät mit einer Luftfördereinrichtung bekannt. Das Beatmungsgerät ist über einen Atemgasschlauch an eine Beatmungsmaske angeschlossen. Die Beatmungsmaske besitzt eine Oberfläche, die antimikrobiellen Eigenschaften aufweist.

In der US 4,677,143 werden verschiedene antimikrobielle Zusammensetzungen beschrieben. Die Zusammensetzungen werden dazu verwendet, im Bereich von medizinischen Geräten Beschichtungen zu erzeugen.

In der US 2003/221691 A1 wird eine Beatmungsmaske beschrieben, die bei einer vom Patienten bemerkten Funktionsstörung durch eine Betätigungseinrichtung gelöst werden kann.

In der EP 1 040 0 874 A2 werden strukturierte Oberflächen beschrieben, die flüssigkeitsabweisenden Eigenschaften aufweisen.

Aus der WO 03/076169 A2 ist ein Spritzgusskörper mit selbstreinigenden Eigenschaften bekannt.

Die WO 02/49980 A1 offenbart die Verwendung von Glas oder Keramik mit einer selbstreinigenden Oberfläche.

In der EP 1 318 228 A1 wird ein Produkt mit einer antiallergischen Beschichtung beschrieben.

Aus der US 2003/0147932 A1 ist ein Produkt bekannt, bei dem in einer funktionalen Beschichtung Mikropartikel enthalten sind.

Die EP 0 933 388 A2 offenbart strukturierte Oberflächen mit einer niedrigen Oberflächenenergie.

Es ist Aufgabe der Erfindung, die hygienische Aufbereitung von von Beatmungsgeräten bestehend aus einer Luftfördereinrichtung, einem Atemgasschlauch und einer Beatmungsmaske, wobei die Luftfördereinrichtung über den Atemgasschlauch an die Beatmungsmaske angeschlossen ist, wobei die Beatmungsmaske als ein modulartiges System ausgebildet ist und ein Bauteil ausgebildet als ein Formkörper aus Kunststoff aufweist, deutlich zu vereinfachen.

Diese Aufgabe wird erfindungsgemäß gelöst durch ein Beatmungsgerät bestehend aus einer Luftfördereinrichtung, einem Atemgasschlauch und einer Beatmungsmaske, wobei die Luftfördereinrichtung über den Atemgasschlauch an die Beatmungsmaske angeschlossen ist, wobei die Beatmungsmaske als ein modulartiges System ausgebildet ist und ein Bauteil ausgebildet als ein Formkörper aus Kunststoff aufweist, wobei mindestens ein Bereich des Formkörpers eine Oberfläche aufweist, die keimabweisende und/oder biozide und/oder antimikrobielle Eigenschaften aufweist, und wobei
- zumindest eine Oberfläche des Formkörpers aus dem Material Polysilikon oder Polysiloxan gefertigt ist, das selbstreinigende Eigenschaften aufweist,
- die Oberfläche des Formkörpers mindestens bereichsweise mit einer Oberflächenbeschichtung versehen ist, wobei die Oberfläche mindestens teilweise oder abschnittsweise eine strukturierte Oberfläche sowohl mit regelmäßigen wie mit unregelmäßigen Erhebungen ist, und
- die Oberfläche zumindest eine festverankerte Lage von Mikropartikeln aufweist, welche Erhebungen bilden und die Erhebungen eine Höhe von 5nm bis 200µm aufweisen.

Darüber hinaus besteht eine zusätzliche Aufgabe der vorliegenden Erfindung darin, ein Verfahren der einleitend genannten Art derart zu verbessern, daß eine funktionelle und zugleich preiswerte sowie langfristig funktionsfähige Ausführungsform bereitgestellt wird.

Diese Aufgabe wird erfindungsgemäß gelöst durch ein Verfahren zur Herstellung eines erfindungsgemäßen definierten Formkörpers, wobei:
- der Formkörper mittels Kunststoffspritzguß hergestellt und anschließend mindestens bereichsweise beschichtet wird, und
- die Oberfläche des Formkörpers nach dem Spritzguß wenigstens abschnittsweise mit keimabweisenden Partikeln versehen wird, wobei ein Partikel aufweisendes Lösungsmittel durch Aufsprühen, Aufrakeln, Auftropfen oder durch Eintauchen aufgebracht wird.

Grundsätzlich wird durch die Verwendung von oberflächenbeschichteten Komponenten in medizintechnischen Geräten ein äußerst weiter konstruktiver Freiraum eröffnet. Die jeweils gewünschten funktionellen Eigenschaften können durch die Oberflächenbeschichtung unabhängig vom Material des Grundkörpers bereitgestellt werden. Die funktionellen Eigenschaften können beispielsweise die bereits erwähnten keimtötenden Eigenschaften sein, die funktionellen Eigenschaften können sich aber auch auf Gleiteigenschaften, Reibungseigenschaften, Oberflächengestaltungen oder Oberflächenhärten beziehen.

Entsprechend der jeweils vorgegebenen funktionellen Eigenschaft wird die Oberflächenbeschichtung des Grundkörpers gewählt und das Basismaterial des Grundkörpers kann unabhängig von der gewünschten funktionellen Eigenschaft der Oberfläche und der mechanischen oder statischen Randbedingungen festgelegt werden. Beispielsweise ist es möglich, bei hohen mechanischen Belastungen einen harten Grundkörper mit einer weicheren Oberflächenbeschichtung zu versehen oder einen weichen und elastischen Grundkörper mit einer keimabweisenden funktionellen Oberfläche auszustatten. Gegebenenfalls werden die gewünschten Oberflächenbeschichtungen unter Verwendung geeigneter Zwischenschichten als Haftvermittler auf die Grundkörper aufgebracht.

Der Formkörper ist insbesondere für Geräteteile, Geräteoberschalen, Geräteinnenteile, Gerätezubehörteile, Gerätekomponenten, Abformmaterialien, Füllungsmaterialien für medizintechnische Geräte und/oder medizinische Produkte ausgebildet und dadurch gekennzeichnet, dass der Formkörpers wenigstens abschnittsweise Keimansiedelungen abweisend ausgebildet ist, sodass ein Ansiedelung von Keimen verhindert wird. So wird auch überraschend einfach erreicht, dass die Infektionsgefahr auch bei immunsuprimierten Nutzern reduziert ist.

Beispielsweise ist der Formkörper im wesentlichen als Geräteteil, Geräteoberschale, Geräteinnenteil, Gerätezubehörteil, Gerätekomponente, Luftanfeuchter, Vernebler, Medikamentenzerstäuber, Beatmungsgerät, Lufteingangsfilter, Schalldämpfer, Luftweg im Gerät, Filter, Beatmungsmaske, Beatmungsschlauch, Notfallbeatmungsgerät, Absaugeinrichtung, Absaugschlauch, Auffangbehälter von einer Absaugeinrichtung oder für als Defibrillatorgehäuse oder -gehäuseteil ausgebildet.

Vorzugsweise ist ein derartiger Formkörper eines medizintechnischen Geräts wenigstens teilweise und/oder abschnittsweise aus Kunststoff gefertigt. Dabei werden häufig verschiedene Kunststoffe verwendet. Die Kunststoffe erfüllen unterschiedliche Funktionen und müssen für die auszuübende Funktion bestmöglich geeignet sein. So sind die Kunststoffe für beispielsweise ein Beatmungsgerät mit den Komponenten Eingangsfilter - Beatmungsgerät - Ausgangsfilter - Patientenschlauch - Filter - Patientenkontaktstelle jeweils für den Einsatzzweck optimiert.

Als Kunststoffe sind alle bekannten Kunststoffe denkbar. Bspw. seien hier genannt: Polyethylene, Polypropylene, Polyvinylchloride, Polystyrole, Polycarbonate, Cellophane, Celluloseacetate, Polyelofine, fluorierte Kohlenwasserstoffe (Teflon), Polyhydroxyethylmethacrylate (PHEMA) (Hydron), Polymethylmethacrylate (PMMA), Polysiloxane, Polyether, Polyester, Polyacetale, Polyvinyle, Polyethersilikone, Polyurethane, Natürliches und synthetisches Gummi, Silikon, Latex, ABS-Harz, Acrylharze, Triacetate, Vinylide und Reyon.

Ferner können alle zum Spritzgießen von Spritzgusskörpern geeigneten Polymere eingesetzt werden. Bevorzugt werden als Materialien für das Spritzgießen Polymere oder Polymerblends eingesetzt, die ein Polymer auf der Basis von Polycarbonaten, Polyoxymethylenen, Poly(meth)acrylaten, Polyamiden, Polyvinylchlorid, Polyethylenen, Polypropylenen, aliphatischen linearen- oder verzweigten Polyalkenen, cyclischen Polyalkenen, Polystyrolen, Polyestern, Polyethersulfonen, Polyacrylnitril oder Polyalkylenterephthalaten, Poly(vinylidenfluorid), Poly(hexafluorpropylen), Poly(per-fluorpropylenoxid), Poly(fluoralkylacrylat), Poly(fluoral-kylmethacrylat), Poly(vinylperfluoralkylether) oder andere Polymere aus Perfluoralkoxyverbindungen, Poly(isobuten), Poly(4- methyl-1-penten), Polynorbonen als Homo- oder Copolymer oder deren Gemische, aufweisen. Ganz besonders bevorzugt werden als Material für das Spritzgießen Polymere oder Polymerblends eingesetzt, die ein Polymer auf Basis von Poly(ethylen), Poly(propylen), Polymethylmethacrylaten, Polystyrolen, Polyestern, AcrylnitrilButadien-Styrol Terpolymere (ABS) oder Poly(vinylidenfluorid) aufweisen, wobei die Kunststoffe in Reinform und/oder als Gemisch verwandt werden können.

Neben Kunststoffen können auch Metalle und/oder Keramik und/oder Glas oder beliebige Kombination dieser Materialien, auch mit den oben genannten Kunststoffen, Anwendung finden.

Der Formkörper kann wenigstens teilweise aus einem keimabweisenden Material gefertigt sein. Hierbei wird unter keimabweisend antibakteriell, antimikrobiell, antiviral, fungizid, germizid oder wachstumshemmend verstanden.

Der Formkörper weist wenigstens abschnittsweise keimabweisend ausgebildete Oberfläche auf.

In einer bevorzugten Ausführungsform ist vorgesehen, dass die Oberfläche des Formkörpers wenigstens abschnittsweise durch Verringerung der Oberflächenadhäsion keimabweisend ausgebildet ist. Bakterien und andere Mikroorganismen benötigen zur Adhäsion an einer Oberfläche oder zur Vermehrung an einer Oberfläche Wasser, welches an hydrophoben Oberflächen nicht zur Verfügung steht. Die erfindungsgemäße Oberfläche verhindert das Anwachsen von Bakterien und anderen Mikroorganismen und ist somit bakteriophob und/oder antimikrobiell.

Ein Ansatz ist die Verringerung der Adhäsion von Proteinen auf Oberflächen. Da Zellmembranen von Mikroorganismen gleichfalls aus Proteinbestandteilen aufgebaut sind, wird durch die Adhäsionsverringerung von Proteinen die Anlagerung von Mikroorganismen reduziert.

Hierzu können Polymere aus einem oder mehreren radikalisch polymerisierbaren Monomeren verwendet werden, wobei diese Polymere Gruppen mit permanenter positiver Ladung und Gruppen, die zur Ausbildung einer Bindung an Oberflächen befähigt sind, besitzen.

Alternativ können zwitterionische Gruppen enthaltende Polymere verwendet werden. Die zwitterionische Gruppe ist üblicherweise ein Ammoniumsalz-Ester. Das Polymer, welches diese Gruppe enthält, kann über die freie radikalische Polymerisation von ethylenisch ungesättigten Monomeren zusammen mit einem Monomer, welches eine zwitterionische Gruppe enthält, hergestellt werden.

Ferner können Phosphorylcholingruppen sowie nichtionischen ethoxylierten und propoxylierten Surfaktanten verwendet werden.

Außerdem können biologische Polymere und synthetische Surfaktanten verwendet werden, um die Adhäsion gewisser Bakterienspezies durch die Erzeugung hydrophober Oberflächen zu verringern.

Es können auch antiadhäsive Oberflächen durch Beschichtungen mit Hyaluronderivaten verwendet werden oder Polymere mit Wiederholungseinheiten mit polaren oder polarisierbaren, modulierenden und hydrophoben Fluoridenthaltenden Gruppen.

Besonders vorteilhaft ist es, speziell modifizierte Kunststoffe zu verwenden, die adhäsionsverringernde Substanzen in einer solchen Menge aufweisen, dass die Adhäsion von Mikroorganismen auf deren Oberfläche um mindestens 50% geringer ist als bei nicht-modifizierten Kunststoffen, und/oder dass sie biozide Substanzen in einer solchen Menge aufweisen, dass mindestens 60% der verbleibenden Mikroorganismen innerhalb von 24 Stunden abgetötet werden.

Es ist vorgesehen, dass die Oberfläche des Formkörpers durch Abtötung und/oder Wachstumshemmung von Mikroorganismen keimabweisend ausgebildet ist. Mikroorganismen sind dabei aus folgenden Gruppen ausgewählte Organismen: Bakterien, Viren, Pilze, Algen, Pflanzen, Tiere, Hefen, Einzeller, Parasiten, Mehrzeller. Zur Abtötung oder Wachstumshemmung können antibakterielle, antimikrobielle, anti-virale, fungizide, germizide, oder wachstumshemmende Substanzen aus allen geeigneten chemischen Gruppen verwendet werden.

In einer weiteren Ausführungsform ist vorgesehen, dass die Oberfläche des Formkörpers als Wirkstoff Biozide aufweist.

Biozide können beispielsweise auf folgenden Gruppen ausgewählt werden: Ciprofloxacin-HCl (Bayer AG, Leverkusen), Ciprofloxacin-Betain, 2-n-Octyl-4-isothiazolin-3-on, 2,3,5,6-Tetrachloro-4-(methylsulphonyl)pyridin, N-Trichloromethyl-thiophthalimid, Chlorhexidin, Triazine, Methylthio-S-Triazine, Bromopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen, langkettige Bisphenolester (US 3,427,345; 3M), Natriumfluorid in Kombination mit Dodecylamin oder anderen organischen Aminen, Benzalkoniumchlorid, Cetylpyridiniumchlorid, 4-Chlor-2-(2,4-Dichlorophenoxy)phenol (Triclosan), Oxiranyltriazolinthione komplexe oder einfache Fluoride wie SnF2, KZnF3, ZnSnF4, Zn(SnF3)2, Kalium oder Zirkonhexafluorotitanat, N-Oxide von gesättigten N-haltigen Heterocyclen substituiert durch Quinoloncarboxylsäuren oder Napthyridoncarboxylsäuren (EP-0 828 715-A1),Monomer Methacryloyloxydodecylpyridiniumbromid (MDPB) in Compositmaterialien, 10,10-Oxybisphenoxyarsin (OBPA), 2-n-Octyl-4-isothiazolin-3-on (OIT), 2,3,5,6-Tetrachloro-4-(methylsulphonyl)pyridin (TCMP) und N-Trichloromethylthiophthalimid (NCMP), Biguanide: -NH-C(NH)-NH-C(NH)-NH- vor allem Polyhexanid und Chlorhexidine, wobei die Biguanide über die Stickstoffatome mit einem Kunststoffpolymer verbunden sind, Polyoxialkyl-Diamin-Biguanide, Biguanid-Polymere mit Isocyanaten oder Epoxiden quervernetzt (nehmen biozide Silber-Salze auf), Quartätres Salz + Antibiotikum, Poly N-halamin, 2-alkyl or 2-aralkylbenzisothiazolin-3-one derivative, Mischung von bioziden in Weichmachern unter Mitverwendung von Zinkoxid, Weichmacher: Adipinsäure- Ester, Phosphorsäure-Ester, Phthalsäure-Ester, Sebazinsäure-Ester, Sulfonsäure- Ester, Trimellitsäure-Ester und Adipinsäure-Polyester, vorzugsweise Phthalsäure- Ester (Unimoll TM , DOP, DIDP, etc.) und Adipinsäure-Polyester Ultramoll TM etc.), insbesondere Kombinationen von Unimoll TM und Ultramoll TM, Halo-Pure TM, N-Halamin, Substanzen, die Hg, Sn, Pb, Bi, Od, Or, Ti, Ag, Cu, Zn, Ni, Pt, Sn, As, Pb, Cd, Cr freisetzen (MicroFree TM AMP, DuPont), beispielsweise Kupferoxide oder Zinksilikate, aber auch die freien Metalle. Silberhalogenide, -sulfate, -carbonate, organische Silber-salze, Antibiotisch wirksames Metallion an Ionenaustauscher gebunden: Zeolithe (A, X, Y and T zeolite), mordenite, sodalite, analcim, chlinoptilolite, erionite und amorphe aluminumsilicate, nicht-zeolitische Trägeroxide aus den Gruppen IIa, IIIa, IIb, IIIb des Periodensystems der Elemente: magnesium, calcium, strontium, barium, boron, aluminum, gallium, indium, zinc, scandium, yttrium and lanthan, cerium oder Mischungen daraus (DE 4425278), AgION antimicrobial (EnsingerGmbH, Nufringen): Zeolith mit großen Porenvolumina bindet Silberionen. Durch Ionenaustauschprozesse setzt das Zeolith die Silberionen frei und bindet dafür Kationen der Umgebung, Mischung aus Siliziumoxid, Aluminiumoxid, Silber oder Silberoxid (insbesondere für Latex und Silikon) DE 4404680, Pyrithionsalz mit Metall, homogene Mischungen von Kalzium-Phosphat und Silber. Ferner können diese genannten Substanzen auch in beliebiger Kombination verwendet werden.

Als adhäsionsverringernde Substanzen und Methoden zur Herstellung adhäsionsverringerter Kunststoffe bzw. Oberflächen eignen sich beispielsweise solche auf Phosphorylcholinbasis oder Phosphorylethanolaminbasis oder auch solche auf der Basis von Polyestern aus Einheiten, die von Glycerophosphorylcholin oder Glycerophosphorylethanolamin abgeleitet sind und polyfunktionellen Säuren oder deren Derivaten. Die adhäsionsverringernden Substanzen werden in Mengen von ca. 0,05 bis 50 Gew.-Teilen, vorzugsweise 0,1 bis 20 Gew.-Teilen, pro 100 Gew.-Teile Gesamtmasse eingebracht oder als Oberflächenbeschichtung aufgebracht.

Die modifizierten Formkörper für medizintechnische Geräte weisen adhäsionsverringernde Substanzen in einer solchen Menge auf, dass die Adhäsion von Mikroorganismen auf deren Oberfläche um mindestens 50% geringer ist als bei nicht-modifizierten Kunststoffen, und/oder dass sie biozide Substanzen in einer solchen Menge aufweisen, dass mindestens 60% der verbleibenden Mikroorganismen innerhalb von 24 Stunden abgetötet werden.

Diese modifizierten Formkörper haben vorzugsweise adhäsionsverringernde Substanzen inkorporiert. Ferner sind deren Oberflächen durch adhäsionsverringernde Substanzen modifiziert.

Außerdem sind vorzugsweise biozide Substanzen inkorporiert, wobei als biozide Substanzen Silber, Silberionen freisetzende Substanzen, Kupfer, Kupferionen freisetzende Substanzen, Zink oder Zinkionen freisetzende Substanzen inkorporiert wurden.

Zur Herstellung modifizierter Kunststoffe werden die adhäsionsverringernden und bioziden Substanzen in die Kunststoffe eingebracht.

In einer weiteren, bevorzugten Weiterbildung ist vorgesehen, dass die Oberfläche des Formkörpers als Wirkstoff Silber und/oder silberhaltige Verbindungen aufweist.

Folgende Tabelle gibt einen Überblick über Eigenschaften verschiedener Silberwirkstoffe.

| Eigenschaften | SilberZeolith | SilberGlaskeramik | Nanosilber |
|---|---|---|---|
| Agens | Ag⁺ | Ag⁺ | Ag⁺ |
| Depot | Ag⁺ | Ag⁺ | Ag⁰ |
| Matrix | Zeolith | Glaskeramik | |
| Partikelgröße | 800-1000 nm | 800 nm | 5-50 nm |
| Beeinträchtigung mechanische Stabilität | ja | ja | keine |
| Lichtempfindlichkeit & Nachdunkelung | ja | ja | keine |
| Depot-Inaktivierung (SH-Rg., Halogenide) | stark | stark | keine |
| Kontrollierbarkeit Ag⁺-Freisetzung Ag⁺-Freisetzung | schlecht | schlecht | sehr gut |

Bspw. ist IONPURE (TM) ist ein Nanosilber-Additiv, welches Kunststoff-Produkten, Kunstfasern, Kompositen, Silikonprodukten, Lacken und Beschichtungen antibakterielle und fungizide Eigenschaften verleiht. Silberionen in einer Glasmatrix sind die Wirkkomponenten. Im Gegensatz zu Standard-Antibiotika sind Bakterien nicht in der Lage, Resistenzen gegen Silberionen zu bilden. Die Einbindung von Silberionen in einer Glasmatrix verhindert unerwünschte Reaktionen, bekannt von anderen Additiven auf Basis von Silberionen, die zum Vergrauen führen.

Die erforderliche Menge von 0,2 - 0,5% IONPURE hat unwesentliche Auswirkungen auf die physikalischen Eigenschaften der Kunststoffprodukte.

Die antimikrobielle Wirkung ist über Jahre hinweg sichergestellt.

Alternativ können kleinste Silberteilchen in den Hohlräumen der Molekülketten von Polymeren verteilt werden. Die Elastizität wird hierbei nicht beeinträchtigt, die biologische Verträglichkeit des Materials ist gewahrt, und die Fähigkeit, Keime abzutöten, bleibt über viele Monate erhalten.

Wenn als Wirkstoff der antimikrobiellen Kunststoffe Silber-ionen verwendet werden, kann als Trägermaterial der Silber-ionen ein Zeolith verwendet werden, ein natürlich vorkommendes, extrem stabiles Mineral. Das Kristallgitter des Zeoliths besitzt große Porenvolumina, so können viele Silberionen gebunden werden. Durch Ionenaustauschprozesse setzt das Zeolith die Silberionen frei und bindet dafür Kationen der Umgebung wie Natrium, Kalium und Calcium. Durch diese kontinuierliche Freisetzung der Silberionen wird eine hohe und langanhaltende Wirkung der antimikrobiellen Kunststoffe erzielt. Der Wirkstoff ist beständig gegen Chemikalien mit pH-Werten von 3 bis 10 sowie gegen Temperaturen bis zu 800°C. Dass das Compound antibakteriell und wachstumshemmend gegenüber Bakterien, Hefen, Schimmel und Pilzen wirkt, konnte bei mehr als 25 Mikrobenstämmen nachgewiesen werden. Dazu gehören z.B. Kolibakterien, Salmonellen und Staphylokokken.

Ein weiteres antibakteriell wirksames Polymere auf Basis nanoskaliger Silberpartikel ist "Polygiene(TM)" (Perstorp, Schweden); eine duroplastische Formmasse mit antiviralen und antibakteriellen Eigenschaften.

Silberpartikel können in Größen bis hinab zu fünf Nanometer Durchmesser hergestellt werden. Dafür wird das Edelmetall im Vakuum verdampft und auf einer Oberfläche wieder kondensiert. Normalerweise erreicht man so Partikelgrößen zwischen 50 und 100 Nanometern, auf fließenden Flüssigkeitsfilmen sind jedoch auch fünf Nanometer erreichbar. Die kondensierten Partikel sind porös und wegen ihrer großen Oberfläche besonders aktiv.

Das Silber ist außerdem hochrein und nicht wie bei chemischen Produktionsprozessen mit anderen Stoffen verunreinigt. Die Nanopartikel werden fein verteilt in Polymere eingearbeitet oder als dünne Beschichtung aufgebracht.

Das Nanosilber wird im Batch verteilt. Im fertigen Produkt wird Nanosilber von der Oberfläche, durch Gebrauch, weggewischt. Silber wird fortlaufend durch Wasser an die Oberfläche gebracht. Die antibakteriellen Eigenschaften bleiben über Monate erhalten.

In einer weiteren, bevorzugten Ausführungsform ist vorgesehen, dass die Oberfläche als Wirkstoff ein antimikrobiell wirksames Polymere aufweist.

Bevorzugt werden zur Herstellung der antimikrobiellen Polymere Stickstoff und Phosphorfunktionalisierte Monomere eingesetzt, insbesondere werden diese Polymere aus mindestens einem der folgenden Monomere hergestellt: Methacrylsäure-2-tert.-butylaminoethylester,Methacrylsäure-2-diethylaminoethylester, Methacrylsäure-2-diethylaminomethylester, Acrylsäure-2-tert.-butylaminoethylester, Acrylsäure-3-dimethylaminopropylester, Acrylsäure-2-diethylaminoethyl-ester, Acrylsäuredimethylaminoethylester, Dimethylaminopropylmethacrylamid, Diethylaminopropylmethacrylamid, Acrylsäure-3-dimethylaminopropylamid, 2-Methacryloyloxethyltrimethylammoniummethosulfat, Methacrylsäure-2-diethylaminoethylester, 2-Methacryloyloxyethyltrimethylammoniumchlorid, <RTI 3-Methacryloylaminopropyltrimethylammonium- chlorid, 2-Methacryloyloxyethyl-trimethylammoniumchlorid, 2-Acryloyloxyethyl-4-benzoydi-methylammoniumbromid,2-Methacryloyloxyethyl-4-benzoyl-dimethylammoniumbromid, Allyltriphenylphosphoniumbromid, Allyltriphenylphosphoniumchlorid, 2-Acrylamido-2-methyl1-propansulfonsäure,2-Diethylaminoethylvinylether, 3-Amino-propylvinylether.

Die antimikrobiell zu versehende Oberfläche kann dabei vollständig oder teilweise mit den Polymeren bedeckt sein.

Es ist möglich, das antimikrobielle Polymere nur anzuschmelzen und/oder nur so wenig auf die Oberfläche aufzubringen, das ggf. eine Kornstruktur des antimikrobiellen Polymeren erhalten bleibt. Es wird so eine Strukturierung der Oberfläche erreicht.

In einer weiteren, bevorzugten Weiterbildung ist vorgesehen, dass die Oberfläche des Formkörpers hydrophil ausgebildet ist. Die Hydrophilierung der antimikrobiell ausgerüsteten Oberflächen bei oder oberhalb der Glastemperatur des antimikrobiellen Polymeren kann durch Kontakt mit Wasser oder Säuren, insbesondere verdünnten organischen oder Mineralsäuren, erfolgen. Die Anreicherung hydrohiler Gruppen, die oftmals Bestandteil antimikrobieller Polymere an der Grenzfläche des beschichteten Substrates sind, begünstigt die antimikrobielle Wirkung noch zusätzlich.

Der Formkörper kann auch eine keimabweisende fotokatalytische Oberfläche aufweisen. Dabei dringen Energieteilchen des Lichtes in den Halbleiter Titandioxid ein und stoßen irgendwann an die Oberfläche. Treffen sie dort auf anhaftendes organisches Material, dann erfolgt ein Elektronenaustausch, das organische Material wird oxidiert. Dabei entsteht als Zwischenprodukt Wasserstoffperoxid (H2O2), das die oxidierende Wirkung noch zusätzlich verstärkt. Fettfilme, Krankheitskeime und andere organische Verunreinigungen werden in Wasser, Kohlendioxid und (wenn vorhanden) Stickstoff zerlegt, also kalt verbrannt. An der Oberfläche des Titandioxids ändert sich dadurch nichts, sie wirkt als Katalysator.

Eine fotokatalytische Beschichtung, die nur aus einem einzigen Nanokomposit besteht, kann auf allen denkbaren Materialien, darunter auch Kunststoffen, so einfach aufgetragen werden wie eine normale Lackierung. Der Lack entsteht bspw. im sogenannten Sol-Gel-Prozess: Organische Lackstrukturen konzentrieren sich zunächst selbsttätig über dem Substrat und bilden eine feste chemische Bindung zum Grundmaterial. Über ihnen reihen sich Nanopartikel aus beispielsweise Silizium, zu einer dichten anorganischen Barriere, die die gesamte Beschichtung nebenbei abriebfest macht. Durch diese kommen nun Milliarden von Nanopartikeln aus Titandioxyd, die sich darüber an der Oberfläche massieren, nicht mit der organischen Schicht, die sie zersetzen würden, in Kontakt. Schließlich werden die Titanpartikel, die zunächst für den Trocknungsprozess beschichtet sind, zur Schichtoberfläche auch noch oxidiert, so dass nun das blanke Titandioxid seine Wirkung entfalten kann.

Der Lack braucht aufgrund dieses Reifungsprozesses einige Zeit, bis er seine volle fotokatalytische Wirkung entfaltet. Die fotokatalytische Wirkung ist deutlich stärker als bei bisherigen Beschichtungen, aus zwei Gründen: Das fotokatalytische Titandioxid liegt in Form von Milliarden Nanopartikeln vor. Wegen der Winzigkeit der Partikel stoßen die angeregten Elektronen und Löcher bei ihrer Bewegung durchs Material viel öfter an die Oberfläche, wo sie dann katalytisch aktiv werden. Hinzu kommt, dass die Masse der Nanopartikel insgesamt eine viel größere Oberfläche hat, als eine kontinuierliche Beschichtung. Dadurch wird auch die aktive Fläche viel größer.

Um die selbstreinigende Wirkung anzustoßen, genügt auch wenig Beschichtungsmaterial. Bei Verschmutzung spielt dabei eine abgelagerte organische Fettschicht eine tragende Rolle, die nicht nur die Oberfläche verschmutzt, sondern auch bewirkt, dass anorganische Staubpartikel an der Oberfläche kleben bleiben. Diese Fettschicht wird fotokatalytisch abgebaut, so dass auch Staubpartikel viel weniger haften. Weil die Titanoberfläche auch noch hydrophil ist, haften sie noch weniger und werden beispielsweise mit Wasser einfach abgewaschen.

Der Formkörper kann eine keimabweisende und/oder kratzfeste Oberfläche aufweisen. Schmutzabweisende Beschichtungen sind hoch abriebfest. Diese Antihaft-Beschichtungen eröffnen durch ihre hohe Abriebfestigkeit viele neue Anwendungsbereiche, bei denen nicht nur die Easy-to-clean-Eigenschaft, sondern auch eine hohe mechanische und chemische Beständigkeit gefordert ist. Die Schichten auf Basis der chemischen Nanotechnologie entstehen nicht mehr über den bislang genutzten Sol-Gel-Prozess, sondern durch organische Polymerchemie mit Integration keramischer Nanopartikel.

Kratzfeste Oberflächen kombinieren bspw. den Kunststoff Polyurethan mit Nanotechnologie. Damit lassen sich die überragenden Eigenschaften des vielseitig eingesetzten Materials, wie etwa Witterungsstabilität, Elastizität und Variationsbreite auf Kratzfestbeschichtungen mit integrierten keramischen Nanopartikeln übertragen. Die unsichtbaren Partikel werden an ihrer Oberfläche so modifiziert, dass sie sich in einem Rührprozess chemisch in das Polyurethan-Netzwerk einbinden lassen. Die transparenten Oberflächen eröffnen einer Reihe neuer Anwendungen für kratzfeste Oberflächen.

Es ist vorgesehen, dass der Formkörper eine keimabweisende selbstreinigende Oberfläche aufweist.

Für selbstreinigende Oberflächen ist insbesondere ist daran gedacht, die Oberflächen leicht mit Wasser reinigen zu können. In welchem Grad ein wassertropfen eine Oberfläche benetzt hängt vom Verhältnis zw. Energieverbrauch für eine Oberflächenvergrößerung und Energiegewinn durch Adsorption ab.

Auf rauen oder hydrophoben Oberflächen entsteht nur wenig Energie durch Adsorption, der Tropfen nimmt annähernd Kugelform an und kann "abrollen". Überrollt ein solcher Tropfen nun einen Schmutzpartikel auf einer rauen Oberfläche, so reduziert sich die Oberfläche des Tropfens relativ zur Luft, die Adsorptionsenergie nimmt zu, und der Partikel haftet an dem Tropfen und wird mitgezogen.

Auf einer glatten Oberfläche dagegen zerfließt der Wassertropfen partiell und kann den Partikel nicht mitnehmen, da dieser durch die Adhäsionskräfte zwischen Partikel und glatter Oberfläche stärker haftet, als auf einer rauen Oberfläche.

Gemäß der Erfindung wird daher vorgeschlagen, die Oberflächen rau und hydrophob zu gestalten. Dabei kann es sich um eine Oberfläche mit einer künstlichen Oberflächenstruktur aus Erhebungen und Vertiefungen handeln, die selbstreinigende Eigenschaften aufweist. Bevorzugt sind die Oberflächenstrukturen im nm bis *µ*m Maßstab. Besonders bevorzugt weisen die Oberflächenstrukturen einen im wesentlichen ähnlichen Abstand zueinander auf. Ferner kann die Oberflächenstruktur zusätzlich Materialien mit antimikrobiellen Eigenschaften aufweisen. Außerdem kann die Oberfläche Partikel aufweisen, die mittels eines Trägersystems auf der Oberfläche fixiert sind.

Besonders bevorzugt ist, dass die Partikel aus einer Mischung von hydrophoben Partikeln und Partikeln mit antimikrobiellen Eigenschaften bestehen.

Das Material mit antimikrobiellen Eigenschaften weist zumindest ein antimikrobielles Polymer auf, welches aus zumindest einem Monomeren ausgewählt aus Methacrylsäure-2-tert.-butylaminoethylester, Methacrylsäure-2-diethylamino-ethylester, Methacrylsäure-2-diethylaminomethylester, Acrylsäure-2-tert.-butylaminoethylester, Acrylsäure-3-dimethylaminopropylester, Acrylsäure-2-diethylaminoethylester, Acrylsäure-2-dimethylaminoethylester, Dimethylaminopropylmethacrylamid, Diethylamino-propylmethacrylamid, Acrylsäure-3-dimethylaminopropylamid, 2-Methacryloyloxy-ethyltrimethylammoniummethosulfat, Methacrylsäure-2-diethylaminoethylester, 2-Methacryloyloxy-ethyltrimethylammoniumchlorid, 3-Methacryloylaminopropyl-trimethylammonium-chlorid, 2-Methacryloyloxyethyltri-methylammoniumchlorid, 2- Acryloyloxyethyl-4-benzoyl-dimethylammoniumbromid, 2- Methacryloyloxyethyl-4-benzoyldimethylammoniumbromid, 2-Acrylamido-2-methyl-1-propansulfonsäure, 2-Diethylaminoethylvinylether oder 3-Aminopropylvinylether hergestellt wurde.

Dabei ist zumindest eine Oberfläche des Formkörpers aus Polysilikon oder Polysiloxan gefertigt, die selbstreinigende Eigenschaften aufweisen. Außerhalb der Erfindung kann sie auch aus einem Material, ausgewählt aus Polymeren, wie z. B. den Polyamiden, Polyurethanen, Polyetherblockamiden, Polyesteramiden, Polyvinylchlorid, Polyolefinen, Polymethylmethacrylaten oder Polyterephthalaten sowie Metallen, Fasern, Geweben, Gläsern oder Keramiken gefertigt werden, das selbstreinigende Eigenschaften aufweist.

Bei der Herstellung der Oberflächenstrukturen wird bevorzugt ein Material eingesetzt, welches antimikrobielle Eigenschaften aufweist. Dabei wird die Oberflächenstruktur, die Erhebungen oder Vertiefungen aufweist, vorzugsweise auf der Oberfläche selbst hergestellt.

Hierzu wird die Oberflächenstruktur durch Aufbringen und Fixieren von Partikeln auf der Oberfläche erzeugt. Zum Fixieren wird ein Trägersystem eingesetzt, wobei die Oberfläche, die Partikel und/oder das Trägersystem das antimikrobielle Material aufweisen können.

Als antimikrobielles Material wird ein Polymer eingesetzt, welches aus zumindest einem Monomeren ausgewählt aus Methacrylsäure-2-tert.-butylaminoethylester, Methacrylsäure-2-diethylaminoethylester, Methacrylsäure-2-diethylamino-methylester, Acrylsäure-2-tert.-butylaminoethylester, Acrylsäure-3-dimethylaminopropylester, Acrylsäure-2-diethylaminoethylester, Acrylsäure-2-dimethylaminoethyl-ester, Dimethylaminopropylmethacrylamid, Diethylamino-propylmethacrylamid, Acrylsäure-3-dimethylaminopropylamid, 2-Methacryloyloxyethyltrimethylammoniummethosulfat, Methacrylsäure-2-diethylaminoethylester, 2-Methacryloyloxyethyl-trimethylammoniumchlorid, 3-Methacryloylaminopropyltri-methylammonium-chlorid, 2-Methacryloyloxyethyltrimethyl-ammoniumchlorid, 2- Acryloyloxyethyl-4-benzoyldimethyl-ammoniumbromid, 2-Methacryloyloxyethyl-4-benzoyldimethyl-ammoniumbromid, 2-Acrylamido-2-methyl-1-propansulfonsäure, 2-Diethylaminoethylvinylether oder 3-Aminopropylvinylether hergestellt wurde.

Als Partikel wird eine Mischung aus Partikeln verwendet, die zumindest ein Material, ausgewählt aus Silikaten, dotierten Silikaten, Mineralien, Metalloxiden, Kieselsäuren oder Polymeren aufweisen, mit Homo- oder Copolymerpartikeln ausgewählt aus Methacrylsäure-2-tert.butylaminoethylester, Methacrylsäure-2-diethylaminoethylester, Methacrylsäure-2-diethylaminomethylester, Acrylsäure-2-tert.-butylaminoethylester, Acrylsäure-3-dimethylaminopropylester, Acrylsäure-2-diethylaminoethylester, Acrylsäure-2-dimethylamino-ethylester, Dimethylaminopropylmethacrylamid, Diethylamino-propylmethacrylamid, Acrylsäure-3-dimethylaminopropylamid, 2-Methacryloyloxyethyltrimethylammoniummethosulfat, Methacrylsäure-2-diethylaminoethylester, 2-Methacryloyloxyethyl-trimethylammoniumchlorid, 3-Methacryloylaminopropyl-trimethylammonium-chlorid, 2-Methacryloyioxyethyltrimethyl-ammoniumchlorid, 2- Acryloyloxyethyl-4-benzoyldimethyl-ammoniumbromid, 2- Methacryloyloxyethyl-4-benzoyldimethyl-ammoniumbromid, 2-Acrylamido-2-methyl-1-propansulfonsäure, 2-Diethylaminoethylvinylether oder 3-Aminopropylvinylether.

Dabei ist vorgesehen, dass die hydrophoben Partikel einen mittleren Partikeldurchmesser von 0,05 bis 30 nm aufweisen.

Hingegen weisen die Partikel mit antimikrobiellen Eigenschaften einen Durchmesser von 0,05 bis 2000 nm auf. Diese Partikel bilden eine unregelmäßige Feinstruktur im Nanometerbereich auf der Oberfläche.

In einer bevorzugten Weiterbildung ist vorgesehen, dass der Formkörper eine keimabweisende strukturierte Oberfläche mit regelmäßigen und/oder unregelmäßigen Erhebungen und/oder Vertiefungen im nm und/oder *µ*M Maßstab aufweist.

Vorzugsweise weisen die Oberflächen selbstreinigende- und/oder hydrophile- und/oder oleophobe- Eigenschaften und/oder eine niedrige Oberflächenenergie auf.

Dabei weist die Oberfläche zumindest eine fest verankerte Lage von Mikropartikeln auf, welche Erhebungen bilden. Die Erhebungen weisen eine mittlere Höhe von 20 nm bis 25 *µ*m und einen mittleren Abstand von 20 nm bis 25 *µ*m auf. Bevorzugt ist jedoch eine mittlere Höhe von 50 nm bis 4 *µ*m und/oder ein mittlerer Abstand von 50 nm bis 4 *µ*m.

Dabei sind die Mikropartikel nanostrukturierte Mikropartikel, die eine Feinstruktur mit Erhebungen bilden. Die Mikropartikel werden ausgewählt aus Partikeln von Silikaten, Mineralien, Metalloxiden, Metallpulvern, Kieselsäuren, Pigmenten, Metallen, Polymeren, pyrogenen Kieselsäuren, Fällungskieselsäuren, Aluminiumoxid, Mischoxiden, dotierten Silikaten, Titandioxiden oder pulverförmigen Polymeren und die bevorzugt hydrophobe Eigenschaften aufweisen.

Dabei ist das Flächenextrudat selbst ein Material, ausgewählt aus Polycarbonaten, Polyoxymethylenen, Poly (meth) acrylaten, Polyamiden, Polyvinylchlorid, Polyethylenen, Polypropylenen, aliphatischen linearen- oder verzweigten Polyalkenen, cyclischen Polyalkenen, Polystyrolen, Polyestern, Polyacrylnitril oder Polyalkylenterephthalaten, Poly (vinylidenfluorid), oder andere Polymere aus Poly (isobuten), Poly(4-methyl-1-penten), Polynorbornen als Homo-oder Copolymer, ein Polymer auf der Basis von Polycarbonaten, Polyoxymethylenen, Poly (meth) acrylaten, Polyamiden, Polyvinylchlorid, Polyethylenen, Polypropylenen, aliphatischen linearen- oder verzweigten Polyalkenen, cyclischen, Polyalkenen, Polystyrolen, Polyestern, Polyacrylnitril oder Polyalkylenterephthalaten, Poly (vinylidenfluorid), oder andere Polymere aus Poly (isobuten), Poly(4-methyl-1-penten), Polynorbonen als Homo-oder Copolymer sowie deren Gemische sowie deren Gemische, aufweist.

Ferner weist es selbstreinigende Eigenschaften und durch Mikropartikel gebildete Erhebungen auf, wobei zur Herstellung in die Oberfläche des Flächenextrudates hydrophobe Mikropartikel eingedrückt werden. Die eingesetzten Mikropartikel weisen einen mittleren Partikeldurchmesser von 0,02 bis 100 *µ*m auf.

Ferner werden hierin strukturierte Oberflächen mit einer niedrigen Oberflächenenergie beschrieben.

Es ist bekannt, dass Oberflächen mit einer Kombination aus Mikrostruktur und geringer Oberflächenenergie interessante Eigenschaften aufweisen. Stand der Technik gemäß EP 0 933 380 ist, dass für solche Oberflächen eine Oberflächenenergie von weniger als 20 mN/m erforderlich ist.

Geeignet sind daher strukturierte Oberflächen, die Erhebungen mit einer mittleren Höhe von 10 nm bis 200 *µ*m und einem mittleren Abstand von 10 nm bis 200 *µ*m aufweisen, und deren äußere Form durch eine mathematische Kurven und/oder Funktionen mit einer Symmetrie bezüglich einer Ebene aufweisen.

Geeignete Materialien sind z. B. Gold, Titan, Silizium, Kohlenstoff, Quarzglas, Lithiumniobat, Siliciumnitrid, Hydroxylapatit, PMMA, Silikone, Epoxydharze, Polydioxanon, Polyamid, Polyimid. Die Charakterisierung von Oberflächen bezüglich ihrer Benetzbarkeit kann über die Messung der Oberflächenenergie erfolgen. Diese Größe ist z. B. über die Messung der Randwinkel am glatten Material von verschiedenen Flüssigkeiten zugänglich (D. K. Owens, R. C. Wendt, J. Appl. Polym. Sci. 13, 1741 (1969)) und wird in mN/m (Milli-Newton pro Meter) angegeben. Nach Owens et al. bestimmt, weisen glatte Polytetrafluorethylen-Oberflächen eine Oberflächenenergie von 19,1 mN/m auf.

Eine besonders niedrige Oberflächenenergie ist insbesondere dann notwendig, wenn nicht nur hydrophobes, sondern auch oleophobes Verhalten gefordert ist. Dies ist insbesondere bei nichtfesten, öligen Verschmutzungen der Fall. (Lotus-Effect) (TM)

Zur Herstellung wird die strukturierte, hydrophobe Oberfläche mit Erhebungen und Vertiefungen mit einem Additiv versetzt, dass eine Partikelgröße von 0,0001 bis 20 *µ*m aufweist und einer organischen Matrix, welche zumindest einen thermoplastischen, elastomeren oder duroplastischen Kunststoff aufweist.

Diese Oberfläche weist selbstreinigende Eigenschaften auf, ist kratzfest, abriebfest und/oder anfärbbar. Ferner ist die Glas- oder Kunststoffoberfläche entspiegelt.

Insbesondere wenn die Oberfläche mit hydrophoben Eigenschaften ausgestattet ist, ist sie schwer von Wasser oder wässrigen Lösungen benetzbar und weist damit selbstreinigende Eigenschaften auf, da Verunreinigungen durch bewegtes Wasser entfernt werden können.

Die Vorrichtung mit Oberflächen, die flüssigkeitsabweisende Eigenschaften und Oberflächenstrukturen mit Erhebungen aufweisen, zeichnet sich dadurch aus, dass die Oberflächen vorzugsweise Kunststoffoberflächen sind, in die Mikropartikel direkt eingebunden und nicht über Trägersysteme oder ähnliches angebunden sind.

Die Vorrichtungen selbst können als Material vorzugsweise Polymere auf der Basis von Polycarbonaten, Polyoxymethylenen, Poly(meth)acrylaten, Polyamiden, Polyvinylchlorid (PVC), Polyethylenen, Polypropylenen, Polystyrolen, Polyestern, Polyethersulfonen, aliphatischen linearen- oder verzweigten Polyalkenen, cyclischen Polyalkenen, Polyacrylnitril oder Polyalkylenterephthalaten sowie deren Gemische oder Copolymere, auf. Besonders bevorzugt weisen die Spritzgusskörper als Material ein Material, ausgewählt aus Poly(vinylidenfluorid), Poly(hexafluorpropylen), Poly(perfluorpropylenoxid), Poly(fluoralkylacrylat), Poly(fluoralkylmethacrylat), Poly(vinylperfluoralkylether) oder andere Polymere aus Perfluoralkoxyverbindungen, Poly(ethylen), Poly(propylen), Poly(isobuten), Poly(4-methyl-1-penten) oder Polynorbonen als Homo- oder Copolymer aufweisen. Ganz besonders bevorzugt weisen die Spritzgusskörper als Material ihr die Oberfläche Poly(ethylen), Poly(propylen), Polymethylmethacrylaten, Polystyrolen, Polyestern, Acrylnitril- Butadien-Styrol Terpolymere (ABS) oder Poly(vinylidenfluorid) auf.

Mikropartikel, die die Erhebungen auf der Oberfläche der Vorrichtung bilden, sind vorzugsweise ausgewählt aus Silikaten, Mineralien, Metalloxiden, Metallpulvern, Kieselsäuren, Pigmenten oder Polymeren, ganz besonders bevorzugt aus pyrogenen Kieselsäuren, Fällungskieselsäuren, Aluminiumoxid, Siliziumoxid, dotierten Silikaten, pyrogenen Silikaten oder pulverförmige Polymeren.

Bevorzugte Mikropartikel weisen eine unregelmäßige Feinstruktur im Nanometerbereich an der Oberfläche auf, einen Partikeldurchmesser von 0,02 bis 100*µ*m, besonders bevorzugt von 0,1 bis 50 *µ*m und ganz besonders bevorzugt von 0,1 bis 10*µ*m auf. Geeignete Mikropartikel können aber auch einen Durchmesser von kleiner als 500 nm aufweisen oder sich aus Primärteilchen zu Agglomeraten oder Aggregaten mit einer Größe von 0,2 bis 100 *µ*m zusammenlagern.

Bevorzugte Mikropartikel sind solche Partikel, die zumindest eine Verbindung, ausgewählt aus pyrogener Kieselsäure, Fällungskieselsäuren, Aluminiumoxid, Siliziumdioxid, pyrogenen und/oder dotierten Silikaten oder pulverförmige Polymeren aufweisen.

Es kann vorteilhaft sein, wenn die Mikropartikel hydrophobe Eigenschaften aufweisen, wobei die hydrophoben Eigenschaften auf die Materialeigenschaften der an den Oberflächen der Partikel vorhandenen Materialien selbst zurückgehen können oder aber durch eine Behandlung der Partikel mit einer geeigneten Verbindung erhalten werden können. Die Mikropartikel können vor oder nach dem Aufbringen bzw. Anbinden auf bzw. an die Oberfläche der Vorrichtung bzw. des Spritzgusskörpers mit hydrophoben Eigenschaften ausgestattet worden sein.

Vorzugsweise sind die Oberflächen mit flüssigkeitsabweisenden Eigenschaften hydrophob, wobei das unstrukturierte Material eine Oberflächenenergie weniger als 35 mN/m, bevorzugt von 10 bis 20 mN/m aufweist.

Sollen Produkte aus Polyolefinen lackiert, bedruckt, beschichtet oder verklebt werden, ist eine Vorbehandlung der Formteile notwendig, da auf der unpolaren Oberfläche dieser Kunststoffe Druckfarben oder Klebstoffe nur unzureichend haften. Üblich sind thermische oder nasschemische Verfahren. Die gewünschte Oxidation der Oberfläche erreicht man auch durch eine - elektronische - Plasmabehandlung.

Ein Plasmaverfahren ermöglicht die Behandlung von Polyolefin-Granulaten und -Pulvern, so daß eine spätere Behandlung der Teile entfallen kann. Mittels eines HF-CVD-Verfahrens können sehr dünne Nanolagen beispielsweise aus Polytetrafluoroethylen (PTFE, [Teflon]) auf verschiedenen Substraten abgeschieden werden. So werden unterschiedliche Materialoberflächen mit beliebigen Eigenschaften funktionalisiert.

Der chemische Sol-Gel-Prozeß, in dem Nanomaterialien entstehen, ist eine in der Werkstoffentwicklung bisher wenig genutzte Variante der anorganischen Synthesechemie. Mit ihm lassen sich aus flüssigen Ausgangsprodukten über einen Niedertemeperaturprozeß anorganische oder anorganische-organische Werkstoffe produzieren und in Zusammensetzung und Struktur breit gestalten.

Die Verwendung von Keramiken zur Nanostrukturierung von Oberflächen hat zum Ziel, Eigenschaften bekannter Werkstoffe zu verändern bzw. bekannte Materialien mit neuen Funktionen zu versehen, bspw. durch Prägeprozesse Säulenstrukturen im Bereich von 20 bis zu 300 Nanometern auf Metall und Kunststoffen zu erzeugen. Das führt zu einer Veränderung der Grenzflächeneigenschaften. Die Ausbildung von Halbkugeln mit einem Radius von 250 bis 350 Nanometern auf zum Beispiel Glasoberflächen vermindert deren Lichtreflexion deutlich. Dieser Effekt beruht auf der Schaffung eines kontinuierlichen Übergangs zwischen der umgebenden Luft und der Glasoberfläche, der so nur durch Nanostrukturen erreicht werden kann.

Eine Kombination aus Mikro- und Nanostrukturierung kann einfach und leicht genutzt werden, um die Benetzung von Oberflächen zu verändern.

Das Aufbringen des die Partikel aufweisenden Lösemittels auf die Polymeroberfläche kann z.B. durch Aufsprühen, Aufrakeln, Auftropfen oder durch Eintauchen der Polymeroberfläche in das die Partikel aufweisende Lösemittel erfolgen.

Durch das beschriebene Verfahren können selbstreinigende Polymeroberfläche hergestellt werden, die eine künstliche, zumindest teilweise hydrophobe Oberflächenstruktur aus Erhebungen und Vertiefungen aufweist, die sich dadurch auszeichnen, dass die Erhebungen und Vertiefungen durch an der Polymeroberfläche fixierte Partikel gebildet werden.

Die Partikel können auch als Aggregate oder Agglomerate vorliegen, wobei gemäß DIN 53 206 unter Aggregaten flächig oder kantenförmig aneinander gelagerte Primärteilchen (Partikel) und unter Agglomeraten punktförmig aneinander gelagerte Primärteilchen (Partikel) verstanden werden.

Die beschriebenen Strukturen können z. B. durch ein Spritzgussverfahren in Kombination mit einem durch LIGA-Verfahren hergestellten, konventionellen Spritzgusswerkzeug hergestellt werden. Das LIGA-Verfahren ist ein Strukturierungsverfahren, das auf Grundprozessen der Röntgen-Lihographie, Galvanik und Abformung beruht. Das Verfahren unterscheidet sich von der Mikromechanik dadurch, dass die Strukturen nicht durch einen Ätzprozess im Grundmaterial erzeugt werden, sondern über ein Werkzeug kostengünstig abgeformt werden können. Im vorliegenden Fall dient das LIGA-Verfahren zur Herstellung des Werkzeugs.

Mit diesem Werkzeug wurden Strukturen in Poly(propylen) abgeformt. Anschließend wurde die Form einer UV-Strahlung von 254 nm für zwei Minuten ausgesetzt. Auf die so aktivierten Oberflächen wurde thermisch Fluoralkylacrylat aufgepfropft Durch diese Vorgehensweise wurde die Oberflächenenergie von etwa 28 mN/m auf weniger als 15 mN/m reduziert.

Hierbei ist es nicht nötig, antimikrobielle Polymere in das Substrat mit einzuarbeiten, wie das z. B. bei der Compoundierung von Formmassen geschieht. Ein weiterer Vorteil des Verfahrens ist die ökonomisch effiziente Einsparung von antimikrobiellen Polymeren, die bei einer Compoundierung z. B. wirkungslos in der Matrix der Formmasse verbleiben. Weiterhin kann man auf diese Weise unerwünschte Veränderungen der physikalischen Eigenschaften der Substrate weitgehend ausschließen, da nur eine sehr dünne Schicht an der Oberfläche des Substrates verändert wird. Das erfindungsgemäße Verfahren kann mit anderen Methoden zur Oberflächennachbehandlung problemlos kombiniert werden. So ist z. B. möglich, nach der thermisch unterstützten Auftragung der Polymere eine Hydrophilierung mit Wasser bzw. Säuren durchzuführen.

Die Werkstoffeigenschaften der verschiedenen Kunststoffe werden nur unwesentlich beeinflusst. Kennwerte wie Dauergebrauchstemperatur, Kriechfestigkeit, thermische und elektrische Isolation bleiben erhalten. Das Compound ist unter allen erdenklichen Herstellungs-, Verarbeitungs- und Anwendungsbedingungen einsetzbar. Es kommt bei Halbzeugen aus PEEK, PPSU, POM-C, PET sowie bei Spritzgussteilen, extrudierten Profilen und kalandrierten Platten zum Einsatz.

Die Formkörper können alternativ und/oder ergänzend zu den vorgenannten Eigenschaften die folgenden Eigenschaften aufweisen. Auch ist daran gedacht, daß ein Formkörper zumindest abschnittsweise mehrere Eigenschaften aufweisen kann.
Flammschutz
gleitreibungsarm
Korrosionsschutz
elektrochemisch aktive
reflektionsarm
elektrochrom
photochrom
piezoelektrisch
leitfähig
Kratzschutz
Antireflex

Zur Herstellung von Formkörpern können alternativ und/oder ergänzend zu den vorgenannten Verfahren die folgenden Verfahren angewandt werden.
Plasmaverfahren
Laserverfahren
Sol-Gel-Verfahren
Galvanische Verfahren
Erzeugung von Nanostrukturen durch Selbstorganisation Nanostrukturierung von Materialien und Oberflächen Aufdampfen im Hochvakuum (Elektronenstrahlverdampfen) Aufdampfen im Hochvakuum (Widerstandstiegelverdampfen) Kathodenzerstäubung
CVD-Verfahren (Chemical Vapour Deposition)
PVD (Physical Vapour Deposition)
LIGA-Verfahren
Thermische Oxidation
Mikrogalvanik (Hartlegierungsabscheidungen)
Spritzguß von Mikrokomponenten aus Kunststoffen Vakuumbeschichtung
chemisch galvanischen Beschichtung
lnmould-coating
Pre-coating
Außerdem gehört zur Erfindung ein Verfahren zur Herstellung von Formkörpern.

In den Zeichnungen sind Ausführungsbeispiele der Erfindung schematisch dargestellt. Es zeigen:
Fig. 1 ein medizintechnisches Gerät,

- Fig. 2: ein Gehäuseteil eines medizintechnischen Gerätes,
- Fig. 3: ebenfalls ein Gehäuseteil eines medizintechnische Gerätes,
- Fig. 4: ein Oberflächenprofil,
- Fig. 5 - 10: verschiedene Oberflächentopographien.
- Fig. 11: eine perspektivische Darstellung eines Befeuchters, der zwischen das Beatmungsgerät und einen Beatmungsschlauch einsetzbar ist,
- Fig. 12: ein Beatmungsgerät mit Zuschaltventil zur Bereitstellung einer erhöhten Sauerstoffkonzentration,
- Fig. 13: eine perspektivische Darstellung einer Beatmungsmaske mit Stirnstütze und
- Fig. 14: eine vergrößerte teilweise Querschnittdarstellung von zwei zusammengefügten Bauelementen.

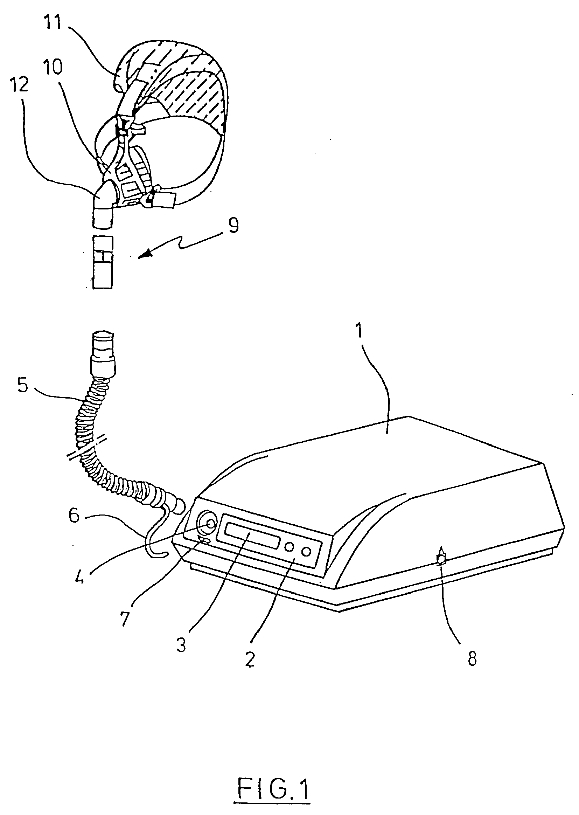

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird eine als Verbindungsschlauch ausgebildete Verbindungsleitung (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung der Verbindungsleitung (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Jedes der o.g. Funktionselemente ist mit jeweils für den Einsatzzweck optimierten modifizierten Kunststoffen ausgestattet, um bestmöglich geeignet zu sein. Die Maske (10) ist beispielsweise mit einer bioziden und einer nichtbeschlagenden Oberfläche versehen. Das Beatmungsgerät (1) ist aus als Gehäuseteile ausgebildeten Formkörpern gebildet, die an den Oberflächen mit einer kratzfesten, bioziden, selbstreinigenden und fotokatalytischen Oberfläche ausgestattet sind. Die Innenflächen des Beatmungsgerätes (1), insbesondere der Schalldämpfer (nicht dargestellt) ist biozid und/oder selbstreinigend. Der Eingangsfilter ist fotokatalytisch und biozid.

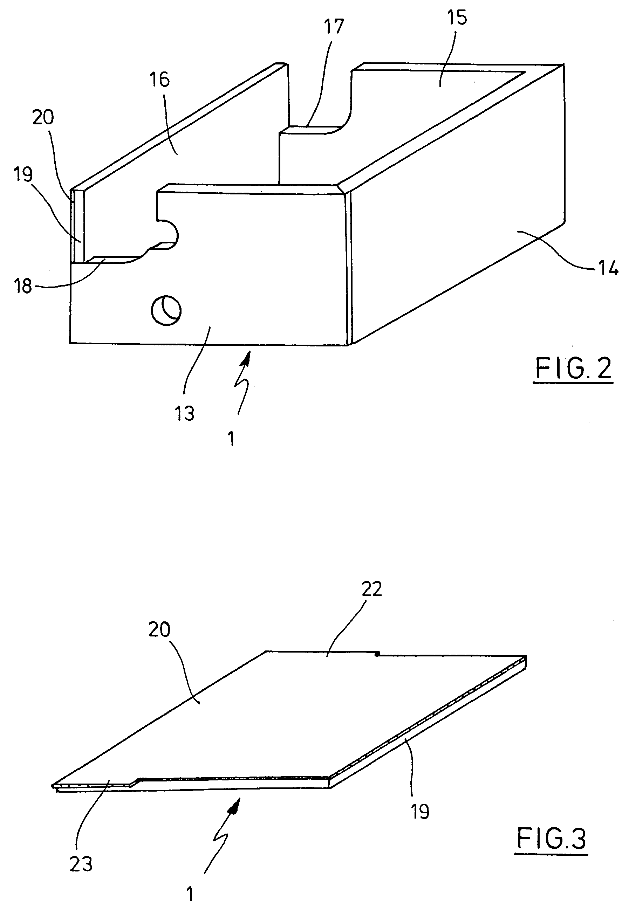

Fig. zeigt ein weiteres Gerätegehäuse (1), das aus plattenförmigen Wandungen (13, 14, 15, 16) besteht. Das Gerätegehäuse (1) ist mit einer Eingangsausnehmung (17) sowie einer Ausgangsausnehmung (18) versehen. Die Wandungen (13, 14, 15, 16) bestehen aus einem Grundmaterial (19), das mit einer Oberflächenbeschichtung (20) versehen ist. Das Gerätegehäuse (1) gemäß Fig. 2 kann beispielsweise als eine Schalldämmbox ausgebildet sein, die zur Aufnahme eines Gebläses oder eines Kompressors vorgesehen ist.

Fig. 3 zeigt ein Deckelteil (21) für das Gerätegehäuse (1) gemäß Fig. 2. Auch das Deckelteil (21) besteht aus einem Grundmaterial (19) und einer Oberflächenbeschichtung (20). Im Bereich der Ausnehmungen (17, 18) des Gerätegehäuses (1) kann das Deckelteil (21) Vorsprünge (22, 23) aufweisen.

Die Oberflächenbeschichtungen (20) können in unterschiedlichen Verfahren hergestellt werden, die teilweise vorstehend bereits beispielhaft erläutert worden. Die Oberflächenbeschichtungen können durch die erläuterten Einbringungen von Partikeln erzeugt werden, es sind aber auch Bedampfungen, Kaschierungen oder Plasmabeschichtungen verwendbar. Ebenfalls können die bereits erläuterten Verfahren zum Aufbringen von flüssigen Beschichtungen in Reinform oder verdünnt mit Lösungsmitteln zum Einsatz kommen. Auch Oberflächenbearbeitungen, beispielsweise unter Verwendung mechanischer Mittel, Laser- oder Elektronenstrahlen sind möglich.

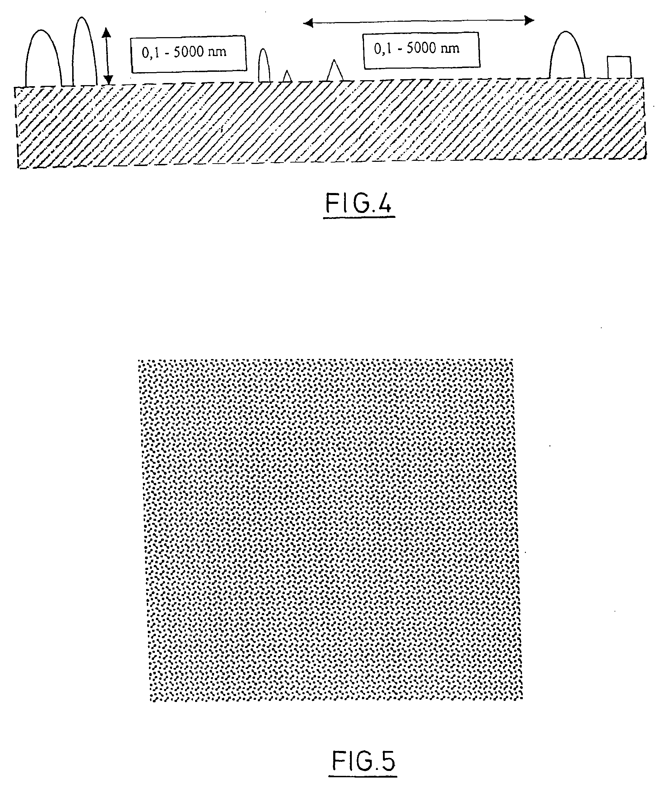

Fig. 4 zeigt einen Abschnitt eines Oberflächenprofils eines modifizierten Formkörpers für ein medizintechnisches Gerät mit Erhebungen verschiedener Form, die eine Höhe bezogen auf den Untergrund von 0,1 nm bis 5000 nm aufweisen. Der Abstand von den einzelnen Erhebungen liegt ebenfalls im Bereich von 0,1 nm bis 5000 nm.

Diese Erhebungen sind in verschiedenen Formen regelmäßige Strukturen bildend auf der Oberfläche angeordnet.

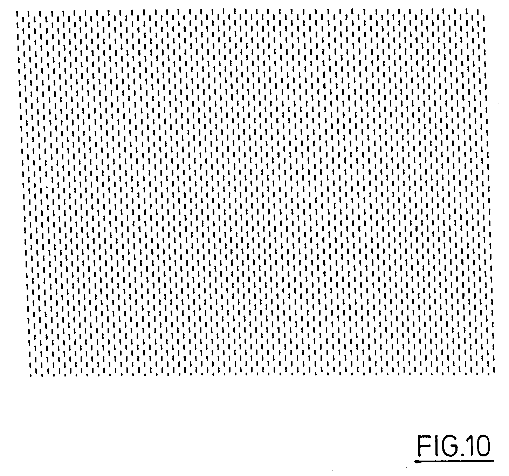

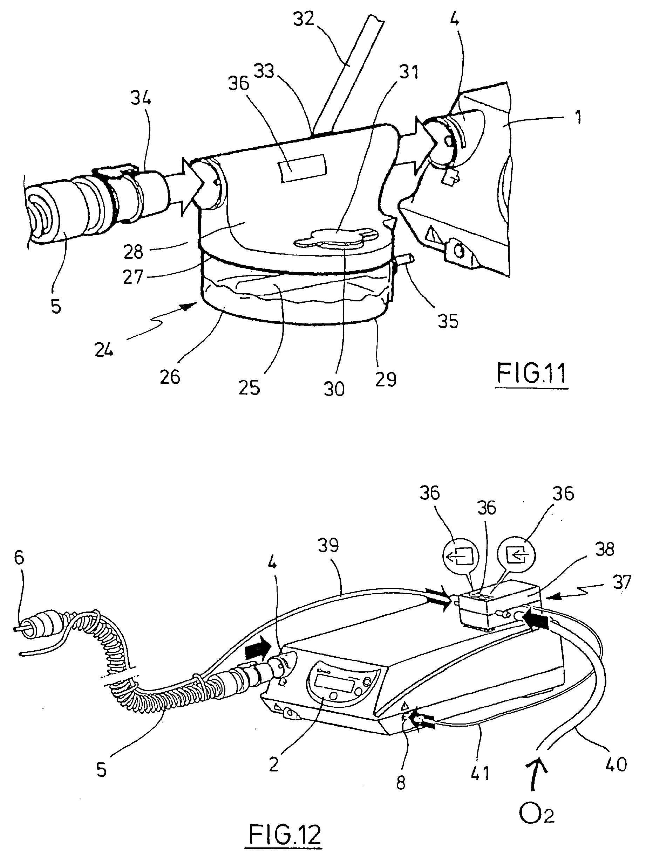

Beispiele umfassen folgende Zubehörteile die im Bereich der Beatmung eingesetzt werden können:
Anfeuchter (Fig. 10), 02-Ventil (Fig. 12), Kopfhaube, Patienten Interface (beispielsweise Maske, Nasal pillows, Tubus) Schlauch, Filter, Halterung, Kupplung, Heizung, Wechselteile, Tasche. Im folgenden wird erläutert, wie die Erfindung zur Verbesserung der genannten Zubehörteile beiträgt.

Bei Beatmungsgeräten wird pro Minute ein Luftvolumenstrom von bis zu 400 l erzeugt. Die Dimensionen eines Beatmungsgerätes, des Patientenschlauches und des Patienteninterface sind im wesentlichen innerhalb enger Grenzen fest vorgegeben. Der Energieaufwand zur Erzeugung des Luftstromes erhöht sich daher mit steigender Strömungsgeschwindigkeit überproportional. Gleichzeitig erhöht sich die Geräuschentwicklung mit steigender Strömungsgeschwindigkeit.

Die Verminderung der Geräuschentwicklung gemessen in 1 Meter Entfernung kann typischerweise wenigstens 5 % oder wenigstens 1 dB(A) betragen. Hinsichtlich einer Verminderung des erforderlichen Energiebedarfes ist insbesondere daran gedacht, daß die Verminderung wenigstens 2 % beträgt. Eine weitere Variante besteht darin, daß die Verminderung eines Zeitaufwandes für eine erforderliche Reinigung wenigstens 10 % beträgt.

Im Hinblick auf auftretende Oberflächenreflexionen kann die funktionale Eigenschaft darin bestehen, daß zumindest für bestimmte Wellenlängen und/oder bestimmte Einfallswinkel eine die Reflektion senkende der die Oberfläche entspiegelnde Wirkung hervorgerufen wird.

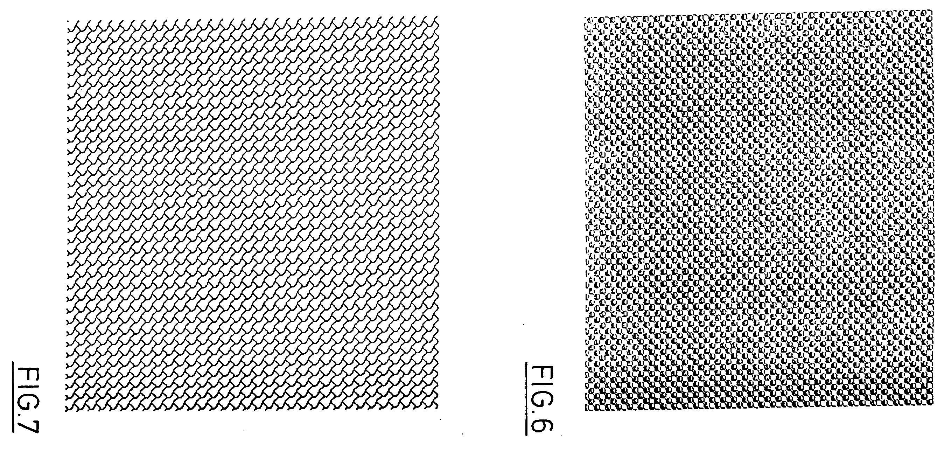

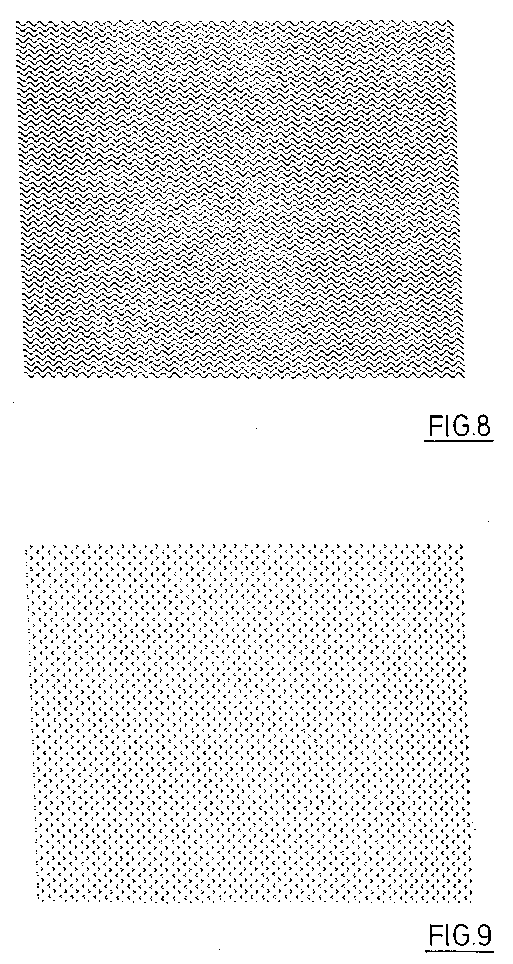

Es wird vorgeschlagen die Reibungskraft der Oberflächen herabzusetzen, um Energie einzusparen und/oder die Geräuschentwicklung zu begrenzen. Die erfindungsgemäßen widerstandsverminderten Oberflächen (Beispiele siehe Fig. 4 - 9) bestehen aus mikroskopisch kleinen Strukturen der Oberfläche beispielsweise Rillen, die bevorzugt parallel zur Strömung des Mediums ausgerichtet sind. Bekannt sind solche Oberflächen aus der Natur, beispielsweise von der Haihaut. Die Oberflächenstrukturen sind so dimensioniert sein, daß sie für das strömende Medium wie eine hydraulisch glatte Oberflächen wirken. Die widerstandsverminderte Wirkung besteht in einer Behinderung der turbulenten Anteile der Strömung an der Wand.

Bevorzugt weisen die Oberflächenstrukturen einen im wesentlichen gleichen Abstand zueinander auf, der im Bereich 100 nm - 200 *µ*m liegt. Besonders bevorzugt im Bereich 5 *µ*m bis 100 *µ*m. Besonders bevorzugt weisen die erfindungsgemäßen Oberflächen eine widerstandsverminderung im Bereich von > 1,0 % auf.

Der luftführende Teil eines Beatmungsgerätes und/oder Schlauches weist erfindungsgemäß wenigstens abschnittsweise eine strukturierte Oberfläche mit regelmäßigen und/oder unregelmäßigen Erhebungen auf und/oder weist eine Oberfläche auf, die die Reibung eines strömenden Mediums vermindert und /oder weist eine strömungsoptimierte Oberfläche auf.

Zur Vermeidung eines Austrocknens der Atemwege wird typischerweise eine Befeuchtung der Atemluft durchgeführt. Da Patienten erwärmte Luft als angenehm empfinden und da durch eine Beheizung beispielsweise mittels eines Heizelements (25) die Luft mehr Wasserdampf aufnehmen kann, erfolgt typischerweise eine Beheizung eines als Flüssigkeitsreservoir (26) verwendeten Wasservorrats des Befeuchtungssystems indirekt und/oder direkt beispielsweise über den metallischen Boden des Wasservorrats beziehungsweise beispielsweise mittels eines Heizstabes (25) direkt im Wasser. Ein Atemgasbefeuchter kann über eine Kopplung (4) extern außenseitig an ein Beatmungsgerät angeschlossen sein und/oder innerhalb eines Beatmungsgerätes angeordnet sein. Aufgrund der einzuhaltenden hygienischen Anforderungen muß der Anfeuchter zum Reinigen demontierbar sein und trotzdem eine ausreichende Abdichtung gegen das Wasser gewährleisten. Der Anfeuchter gliedert sich in ein, im wesentlichen luftführendes, Oberteil (28), welches auch der Verbindung von Beatmungsgerät (1) und Verbindungsschlauch (5) dient, und eine Unterteil (29) das den Wasservorrat aufnehmen kann. Im Bereich des Oberteiles (28) ist eine Einfüllöffnung für Flüssigkeit (30) mit Verschluß (31) angeordnet.

Im Bereich des Anfeuchters kann eine Gasleitung (32) angeordnet sein, die bevorzugt als Druckmeßleitung und/oder Sauerstoffzuleitung ausgebildet ist. Die Gasleitung ist über eine Gaskupplung (33) mit dem Anfeuchter verbunden. Der Anfeuchter ist über einen Anschlußadapter (34) mit einem Verbindungsschlauch (5) koppelbar. Eine Kommunikation mit dem Beatmungsgerät (1) kann über eine vorhandene Steckverbindung (35) zwischen dem Anfeuchter und dem Beatmungsgerät realisiert werden. Im Bereich des Anfeuchters kann eine Anzeige (36) positioniert werden.

Üblicherweise wird eine Dichtung (27) zwischen zwei demontierbaren Teilen des Anfeuchters angeordnet, um den Austritt von Wasser und/oder Atemgas zu verhindern. Insbesondere muß der Anfeuchter von innen regelmäßig gereinigt werden. Typischerweise werden Anfeuchter nach dem Stand der Technik in einer Spülmaschine gereinigt und gegebenenfalls zusätzlich desinfiziert, der Aufwand für die Pflege und die Reinigung ist somit hoch.

Dieser Nachteil wird dadurch behoben, daß der Anfeuchter demontierbar ist und daß die Oberflächen des Anfeuchters wenigstens abschnittsweise die erfindungsgemäßen funktionalen Oberflächen aufweisen. Oberflächen sind Flächen des Anfeuchters, die mit einem Medium wie Luft oder Wasser in Kontakt stehen. Die Oberflächen können hierbei innere Oberflächen des Anfeuchters sein, wie beispielsweise die Innenseite des Wasservorrats und/oder die Innenseite des luftführenden Teils des Anfeuchters, es ist jedoch ebenso die nach außen weisende Oberfläche gemeint.

Kontaktstellen sind Flächen des Anfeuchters und/oder Beatmungsgerätes und/oder Verbindungsschlauches und/oder des Patienteninterface und/oder des Sauerstoffzuschaltventils, die mit einem anderen Formkörper in Kontakt stehen. Die Kontaktstellen können hierbei innere Kontaktstellen eines Formkörpers sein, wie beispielsweise die Kontaktstelle zwischen Unterteil (29) und Oberteiles (28) des Anfeuchters, es ist jedoch ebenso die nach außen weisende Kontaktstelle gemeint wie beispielsweise die Kontaktstelle zwischen Anfeuchter und Beatmungsgerät.

Ein weiteres Anwendungsbeispiel wird nachfolgend in Bezug auf ein Sauerstoffzuschaltventil (37) erläutert. Ein Sauerstoffzuschaltventil (37) ist eine Einrichtung zur Zufuhr von Sauerstoff zu einem Anwender.

Fig. 12 zeigt eine perspektivische Darstellung eines Beatmungsgerätes (1), das mit einer Kopplung (4), einem Bedienfeld (2) versehen ist. Mit der Kopplung (4) ist ein Verbindungsschlauch (5) verbunden, durch den sich eine Druckmeßleitung (6) hindurch erstreckt.

Außenseitig am Verbindungsschlauch (5) ist eine Sauerstoffleitung (39) verlegt, die mit einem SauerstoffZuschaltventil (37) verbunden ist. Das Zuschaltventil (39) ist über eine Versorgungsleitung (40) an eine nicht dargestellte Sauerstoffquelle angeschlossen. Über eine Steuerleitung (41) ist das Zuschaltventil (39) mit einem Schnittstelle (8) des Beatmungsgerätes (1) verbunden.

Bei dem in Fig. 12 dargestellten Beispiel ist das Zuschaltventil (39) außenseitig am Beatmungsgerät (1) angeordnet. Es ist jedoch ebenso daran gedacht, das Zuschaltventil in das Beatmungsgerät zu integrieren.

Bevorzugt weist ein Sauerstoffzuschaltventil (37) wenigstens abschnittsweise keimabweisend ausgebildete Oberfläche auf. Die Oberflächen (38) des Sauerstoffzuschaltventils sind beispielsweise wenigstens abschnittsweise durch Verringerung der Oberflächenadhäsion keimabweisend ausgebildet. Die Oberflächen des Sauerstoffzuschaltventils sind beispielsweise wenigstens abschnittsweise durch Abtötung und/oder Wachstumshemmung von Mikroorganismen keimabweisend ausgebildet. Beispielsweise weisen die Oberfläche des Sauerstoffzuschaltventils Biozide auf. Die Oberfläche des Sauerstoffzuschaltventils weist wenigstens abschnittsweise bevorzugt als Wirkstoff Silber und/oder silberhaltige Verbindungen auf. In einem anderen Ausführungsbeispiel weist die Oberfläche als Wirkstoff ein antimikrobiell wirksames Polymer auf.

Die Oberfläche des Sauerstoffzuschaltventils kann alternativ und/oder zusätzlich wenigstens abschnittsweise hydrophil ausgebildet ist. Die Oberfläche des Sauerstoffzuschaltventils kann wenigstens abschnittsweise alternativ keimabweisend und/oder fotokatalytisch und/oder zusätzlich hydrophil ausgebildet sein. Das Sauerstoffzuschaltventil kann zumindest abschnittsweise eine kratzfeste Oberfläche aufweisen. Das Sauerstoffzuschaltventil kann bevorzugt eine keimabweisende, selbstreinigende und/oder hydrophile und/oder oleophobe und/oder reibungsarme und/oder leitende Oberfläche besitzen.

Ein weiteres Anwendungsbeispiel wird in Bezug auf eine Kopfhaube nachfolgend erläutert. Wie Fig. 1 zeigt, ist eine Beatmungsmaske (10) typischerweise mit einer Kopfhaube (11) im Bereich des Kopfes fixiert. Die Kopfhaube weist üblicherweise mindestens ein Band auf, daß im Bereich seiner der Beatmungsmaske abgewandten Ausdehnungen miteinander verbunden ist und maskenseitig an zumindest zwei Stellen mit der Maske kontaktiert. Die Befestigung der Kopfhaube an der Maske erfolgt üblicherweise über Kontaktmechanismen wie beispielsweise Klips und/oder Haken und/oder Schlaufen. Die Kopfhaube kann zusätzlich durch ein Kinnband ergänzt werden.

Bedingt durch den ständigen Kontakt zum Patienten muß die Kopfhaube gereinigt werden können. Eine mögliche, hygienisch bedeutsame, Verunreinigung muß sicher entfernt werden können. Bei Kopfhauben nach dem Stand der Technik, die Typischerweise in der Waschmaschine gewaschen werden, ist die einwandfreie hygienische Aufbereitung nicht sichergestellt. Dieser Nachteil wird erfindungsgemäß dadurch gelöst, daß die Oberflächen der Kopfhaube und/oder die Oberflächen der Kontaktmechanismen wie beispielsweise Klips und/oder Haken und/oder Schlaufen wenigstens abschnittsweise die erfindungsgemäßen funktionalen Oberflächen aufweisen. Im folgenden Oberflächen der Kopfhaube genannt. Oberflächen der Kopfhaube können auch innere Oberflächen sein, wenn beispielsweise ein grobporiges Gewebe verwandt wird.

Bevorzugt weist die Kopfhaube wenigstens abschnittsweise keimabweisend ausgebildete Oberfläche auf. Die Oberflächen der Kopfhaube sind beispielsweise wenigstens abschnittsweise durch Verringerung der Oberflächenadhäsion keimabweisend ausgebildet. Die Oberflächen der Kopfhaube sind beispielsweise wenigstens abschnittsweise durch Abtötung und/oder Wachstumshemmung von Mikroorganismen keimabweisend ausgebildet. Beispielsweise weisen die Oberfläche der Kopfhaube Biozide auf. Die Oberfläche der Kopfhaube weist wenigstens abschnittsweise bevorzugt als Wirkstoff Silber und/oder silberhaltige Verbindungen auf. In einem anderen Ausführungsbeispiel weist die Oberfläche als Wirkstoff ein antimikrobiell wirksames Polymer auf.

Die Kopfhaube kann bevorzugt eine keimabweisende und/oder selbstreinigende und/oder hydrophobe und/oder oleophobe und/oder fotokatalytische und/oder elektrisch leitfähige Oberfläche besitzen.

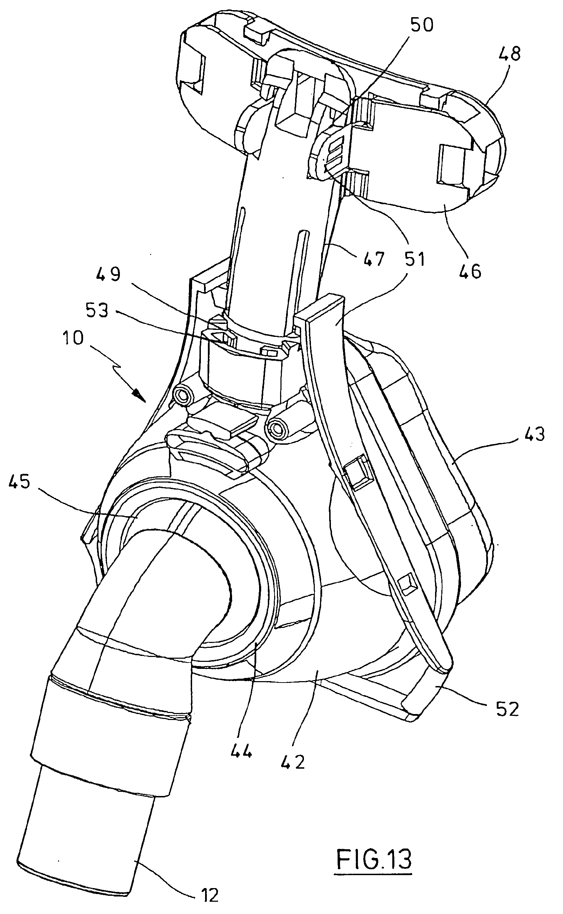

Als nächstes Anwendungsbeispiel wird nachfolgend ein Patienteninterface erläutert. Gemäß dem in Fig. 13 dargestellten Ausführungsbeispiel ist ein Patienteninterface als eine Gesichts-Maske (10) ausgeführt. Üblicherweise ist eine Maske als ein modulartiges System ausgeführt und besteht typisch aus folgenden Bauteilen, wobei diese Aufzählung nicht erschöpfend ist:
Maskenkörper (42) und/oder Maskenwulst (43) und/oder Ausatemsystem (44) und/oder Kupplungselement (12) und/oder Gelenk (45) und/oder Stirnstütze (46) und/oder Halterung der Stirnstütze (47) und/oder Stirnpolster (48) und/oder Befestigungsvorrichtung (52) für eine Kopfbänderung Sicherungsring und/oder Reißleine. Nicht alle einzelnen Bauteile müssen zwingend im Bereich einer Maske angeordnet sein, damit diese funktionsfähig ist.

Der Maskenwulst (43) dient zur Anlage am Gesicht eines Patienten und gewährleistet die erforderliche Abdichtung. Über ein Gelenk ist der Maskenkörper mit einem Kupplungselement (12) verbunden, die zum Anschluß an einen Atemgasschlauch dient. Zur Gewährleistung einer sicheren Positionierung der Atemmaske im Kopfbereich eines Patienten wird eine Stirnstütze (47) verwendet, die mit einem Stirnpolster (48) ausgestattet ist. Die Stirnstütze ist über eine Halterung (53) mit dem Maskenkörper verbunden.

Eine Grob (49) - und Fein (50) - Verstellung für die Stirnstütze dient der Justierung der Masken am Patienten. Die Feinverstellung kann beispielsweise über feine Rastungen an der Stirnstütze bzw. am Stirnpolster erfolgen. Die Grobverstellung erfolgt beispielsweise durch ein Umstecken der Stirnstütze im Bereich der Halterung (53). Mit Hilfe der Grobverstellung rückt der Patient beispielsweise die Stirnstütze in die gewünschte Position und fixiert die Stirnstütze unter Verwendung der Feinverstellung am Stirnpolster in der ausgewählten Position.

Für die Justierung einer Masken finden komplexe Schwenk- oder Schiebemechanismen ebenfalls Verwendung. Die Verstellmechanismen weisen jeweils Bedienflächen (51) für den Anwender auf. Durch eine leichte mechanische Einwirkung eines Anwenders im Bereich der Bedienflächen lassen sich die Masken-Bauteile relativ zueinander bewegen.
Wenn die Beatmungsmaske ohne Stirnstütze verwendet wird, kann in die Steckverbindung ein Blindstopfen eingesetzt werden oder es ist die Verwendung eines Adapters möglich, der eine Kopplung mit der Kopfbänderung ermöglicht. Das Stirnpolster kann bevorzugt von der Stirnstütze lösbar sein. Hierdurch kann eine leichte Austauschbarkeit und/oder getrennte Reinigung erfolgen.

Der Maskenwulst ist Typischerweise relativ zum Maskenkörper abnehmbar. Die Fixierung des Maskenwulstes im Maskenkörper erfolgt beispielsweise durch Einschnitte, Stege, Verdickungen oder Aussparungen.

Im Bereich des Maskenkörpers kann ein Löseelement angebracht werden, die es dem Patienten ermöglicht, mit einem einzigen Zug eine Verbindung zwischen dem Beatmungsschlauch und der Atemgasmaske zu lösen.

Alternativ zu einer Maske werden verschiedene andere Patienten-Interfaces verwandt. Beispielhaft sind folgende genannt: Nasenstopfen (Nasal pillows), Tuben, Tracheostoma, Katheter.

Eine wichtige Voraussetzung für Patienten-Interfaces ist, daß Sie einfach und effektiv zu reinigen sein müssen. Hierzu finden die Oberflächen im Bereich der Masken Verwendung. Im folgenden werden Masken und alle Masken-Bauteile, sowie andere Patienten-Interfaces wie Nasal pillows vereinfacht unter dem Begriff Patienten-Interfaces zusammengefaßt.

Bevorzugt weisen Patienten-Interfaces wenigstens abschnittsweise keimabweisend ausgebildete Oberfläche auf. Die Oberflächen der Patienten-Interfaces sind beispielsweise wenigstens abschnittsweise durch Verringerung der Oberflächenadhäsion keimabweisend ausgebildet. Die Oberflächen der Patienten-Interfaces sind beispielsweise wenigstens abschnittsweise durch Abtötung und/oder Wachstumshemmung von Mikroorganismen keimabweisend ausgebildet. Beispielsweise weisen die Oberflächen der Patienten-Interfaces Biozide auf. Die Oberflächen der Patienten-Interfaces weisen wenigstens abschnittsweise bevorzugt als Wirkstoff Silber und/oder silberhaltige Verbindungen auf. In einem anderen Ausführungsbeispiel weisen die Oberfläche als Wirkstoff ein antimikrobiell wirksames Polymer auf. Besonders bevorzugt sind die Bedienflächen biozid und/oder keimabweisend.

Patienten-Interfaces können bevorzugt wenigstens abschnittsweise eine keimabweisende und/oder selbstreinigende und/oder hydrophobe und/oder oleophobe und/oder fotokatalytische und/oder kratzfeste und/oder beschlagfreie und/oder hautfreundliche und/oder reibungsarme und/oder elektrisch leitfähige Oberfläche besitzen.

Der Maskenwulst und/oder das Stirnpolster und/oder nasal pillows sind bevorzugt keimabweisend und/oder selbstreinigend und/oder hautfreundlich und/oder hydrophob und/oder oleophob.

Der Maskenkörper ist auf seiner dem Patienten zugewandten Seite bevorzugt keimabweisend und/oder selbstreinigend und/oder hydrophob und/oder oleophob und/oder fotokatalytisch und/oder kratzfest und/oder beschlagfrei und/oder hautfreundlich. Der Maskenkörper ist auf seiner dem Patienten abgewandten Seite bevorzugt keimabweisend und/oder selbstreinigend und/oder hydrophob und/oder oleophob und/oder fotokatalytisch und/oder kratzfest. Insbesondere ist daran gedacht Einschnitte, Stege, Verdickungen oder Aussparungen im Bereich von Patienten-Interfaces keimabweisend und/oder selbstreinigend und/oder hydrophob und/oder oleophob und/oder fotokatalytisch und/oder kratzfest auszurüsten.

Auch ist daran gedacht Kontaktstellen der einzelnen Bauteile zueinander und/oder Kontaktstellen zum Patienten keimabweisend und/oder selbstreinigend und/oder hydrophob und/oder oleophob auszurüsten.

Besonders bevorzugt ist daran gedacht Patienten Interfaces im Bereich Ihrer der Luftströmung zugewandten Seite durch geeignete glatte Kunststoffe und/oder lakkierte Oberflächen und/oder beschichtete Kunststoffe und/oder Oberflächen die eine Strukturierung im Nanometer- bis Mikrometer-Maßstab aufweisen derart auszurüsten, daß hierdurch eine reduzierte Reibung erzielt werden kann.

Die Vorteile der können auch im Zusammenhang mit einem Filter angewendet werden. Insbesondere bei Beatmungsgeräten, aber auch bei anderen medizintechnischen Geräten werden vor allem im Ansaugbereich der Luft Filter eingesetzt um Schwebstoffe, Staubteile, Mikroorganismen zurückzuhalten. Mit Hilfe der Filter sollen Verunreinigungen des Geräts und die Kontamination des Patienten vermieden werden. Alternativ und/oder zusätzlich werden auch im Bereich zwischen Gerät und Patient beziehungsweise Anwender Filter eingesetzt, vor allem um hygienische Kontaminationen zu vermeiden. Üblicherweise werden die Filter als Steckfilter auswechselbar eingesetzt. Auch werden sogenannte Kombinationsfilter eingesetzt, die beispielsweise als Grob- und Fein-Filter ausgebildet sein können. Wird ein Filter nicht regelmäßig gereinigt und/oder ausgewechselt, so können Zurückgehaltene Schwebstoffe, Staubteile, Mikroorganismen den Durchströmungswiderstand des Filters heraufsetzen was dazu führt, daß die Leistung des Gerätes abnimmt beziehungsweise Verunreinigungen zum Patienten geleitet werden. Filter nach dem Stand der Technik müssen häufig ausgetauscht werden, was zeit- und kostenintensiv ist.

Es ist daran gedacht, die Filter mit funktionalen Oberflächen auszurüsten. Dadurch wird eine längere Betriebsdauer der Filter ermöglicht, was hilft Kosten einzusparen.

Die Filter sind auf der dem Patienten zugewandten Seite bevorzugt keimabweisend und/oder selbstreinigend und/oder hydrophob und/oder oleophob und/oder fotokatalytisch. Insbesondere ist auch daran gedacht Einschnitte, Stege, Verdickungen oder Aussparungen im Bereich der Fixierung eines Filters keimabweisend und/oder selbstreinigend und/oder hydrophob und/oder oleophob und/oder fotokatalytisch auszurüsten.

Auch ist daran gedacht Kontaktstellen zwischen Filter und Gerät und/oder Kontaktstellen zwischen Filter und Anwender keimabweisend und/oder selbstreinigend und/oder hydrophob und/oder oleophob und/oder fotokatalytisch auszurüsten. Durch die fotokatalytischen Eigenschaften werden anhaftende Teilchen und Mikroorganismen kalt verbrannt.

Bevorzugt ist daran gedacht Filter im Bereich Ihrer der Luftströmung zugewandten Seite durch geeignete glatte Kunststoffe und/oder lackierte Oberflächen und/oder beschichtete Kunststoffe und/oder Oberflächen die eine Strukturierung im Nanometer- bis Mikrometer-maßstab aufweisen derart auszurüsten, daß hierdurch eine reduzierte Reibung erzielt werden kann.
Bevorzugt ist ebenfalls daran gedacht sogenannte HME-Filter (Heat moisture exchange) derart auszurüsten, daß sie einen reduzierten Reibungswiderstand aufweisen und/oder keimabweisend und/oder selbstreinigend und/oder oleophob und/oder fotokatalytisch sind.

Funktionelle Oberflächen erweisen sich auch bei Schläuchen als vorteilhaft. Insbesondere bei Beatmungsgeräten, aber auch bei anderen medizintechnischen Geräten wie beispielsweise Absauggeräten werden vor allem im Bereich einer Verbindung zwischen Anwender/Patient mit dem Gerät Schläuche eingesetzt, um ein Medium zu leiten. Üblicherweise werden die Schläuche als Steckschläuche auswechselbar eingesetzt. Wird ein Schlauch nicht regelmäßig gereinigt und/oder ausgewechselt, so können Zurückgehaltene Schwebstoffe, Staubteile, Verunreinigungen Mikroorganismen den Durchströmungswiderstand heraufsetzen was dazu führt, daß die Leistung des Gerätes abnimmt beziehungsweise Verunreinigungen zum Patienten geleitet werden. Schläuche nach dem Stand der Technik müssen häufig gereinigt und/oder ausgetauscht werden, was zeit- und kostenintensiv ist. Die Reinigung muß häufig und gründlich erfolgen, um Kontaminationen effektiv zu entfernen.

Es ist daran gedacht, die Schläuche mit funktionalen Oberflächen auszurüsten. Dadurch wird eine längere Betriebsdauer der Schläuche bei einem gleichzeitig geringeren zeitlichen Aufwand für die Reinigung ermöglicht, was Kosten einspart.

Die Schläuche sind auf der dem Patienten/Anwender zugewandten Seite bevorzugt keimabweisend und/oder selbstreinigend und/oder hydrophob und/oder oleophob und/oder fotokatalytisch. Insbesondere ist auch daran gedacht Einschnitte, Stege, Verdickungen oder Aussparungen im Bereich der Fixierung eines Schlauches am Gerät und/oder am Patienten keimabweisend und/oder selbstreinigend und/oder hydrophob und/oder oleophob und/oder fotokatalytisch auszurüsten.

Auch ist daran gedacht Kontaktstellen zwischen Schlauch und Gerät und/oder Kontaktstellen zwischen Schlauch und Anwender keimabweisend und/oder selbstreinigend und/oder hydrophob und/oder oleophob fotokatalytisch auszurüsten. Durch die fotokatalytischen Eigenschaften werden anhaftende Teilchen und Mikroorganismen kalt verbrannt.

Bevorzugt ist daran gedacht Schläuche im Bereich Ihrer der Luftströmung/Medienströmung zugewandten Seite durch geeignete glatte Kunststoffe und/oder lackierte Oberflächen und/oder beschichtete Kunststoffe und/oder Oberflächen die eine Strukturierung im Nanometer- bis Mikrometer-maßstab aufweisen derart auszurüsten, daß hierdurch eine reduzierte Reibung erzielt werden kann. Bevorzugt ist ebenfalls daran gedacht, daß Schläuche eine elektrisch leitfähige Oberfläche aufweisen. Bevorzugt weist eine Tasche zur Aufnahme eines Medizingerätes und/oder zur Aufnahme von Zubehör für ein Medizingerät wenigstens abschnittsweise keimabweisend ausgebildete Oberfläche auf. Die Oberflächen der Tasche sind beispielsweise wenigstens abschnittsweise durch Verringerung der Oberflächenadhäsion keimabweisend ausgebildet. Die Oberflächen der Tasche sind beispielsweise wenigstens abschnittsweise durch Abtötung und/oder Wachstumshemmung von Mikroorganismen keimabweisend ausgebildet. Beispielsweise weisen die Oberfläche des Tasche Biozide auf. Die Oberfläche der Tasche weist wenigstens abschnittsweise bevorzugt als Wirkstoff Silber und/oder silberhaltige Verbindungen auf. In einem anderen Ausführungsbeispiel weist die Oberfläche als Wirkstoff ein antimikrobiell wirksames Polymer auf.

Die Oberfläche der Tasche kann alternativ und/oder zusätzlich wenigstens abschnittsweise hydrophil ausgebildet ist. Die Oberfläche der Tasche kann wenigstens abschnittsweise alternativ keimabweisend und/oder fotokatalytisch und/oder zusätzlich hydrophil ausgebildet sein. Die Tasche kann zumindest abschnittsweise eine kratzfeste Oberfläche aufweisen. Die Tasche kann bevorzugt eine keimabweisende, selbstreinigende und/oder hydrophile und/oder oleophobe und/oder reibungsarme und/oder leitende Oberfläche besitzen.

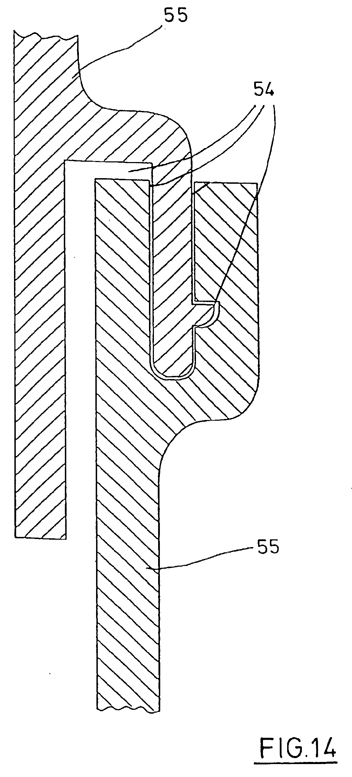

Fig. 14 zeigt eine beispielhafte Ausführungsform von Kontaktstellen (54) der erfindungsgemäße Formkörper (55) für medizinische Geräte. Bevorzugt weisen erfindungsgemäße Formkörper (55) für medizinische Geräte wenigstens abschnittsweise keimabweisend ausgebildete Kontaktstellen (54) auf. Die Kontaktstellen (54) sind beispielsweise wenigstens abschnittsweise durch Verringerung der Oberflächenadhäsion keimabweisend ausgebildet. Die Kontaktstellen sind beispielsweise wenigstens abschnittsweise durch Abtötung und/oder Wachstumshemmung von Mikroorganismen keimabweisend ausgebildet. Beispielsweise weisen die Kontaktstelle des Biozide auf. Die Kontaktstellen weisen wenigstens abschnittsweise bevorzugt als Wirkstoff Silber und/oder silberhaltige Verbindungen auf. In einem anderen Ausführungsbeispiel weist die Kontaktstelle als Wirkstoff ein antimikrobiell wirksames Polymer auf.

Die Kontaktstellen können alternativ und/oder zusätzlich wenigstens abschnittsweise hydrophil ausgebildet ist. Die Kontaktstellen können wenigstens abschnittsweise alternativ keimabweisend und/oder fotokatalytisch und/oder zusätzlich hydrophil ausgebildet sein. Ein Formkörper kann zumindest abschnittsweise eine kratzfeste Kontaktstelle aufweisen. Ein Formkörper kann bevorzugt eine keimabweisende, selbstreinigende und/oder hydrophile und/oder oleophobe und/oder reibungsarme und/oder leitende Kontaktstelle besitzen. Ebenfalls ist daran gedacht, daß die Kontaktstellen alle im Text erwähnten Eigenschaften entweder allein oder in Kombination zumindest abschnittsweise aufweisen können.

Diese modifizierten Formkörper für medizintechnische Geräte weisen adhäsionsverringernde Substanzen in einer solchen Menge auf, dass die Adhäsion von Mikroorganismen auf deren Oberfläche um mindestens 50% geringer ist als bei nicht-modifizierten Formkörpern, und/oder dass sie biozide Substanzen in einer solchen Menge aufweisen, dass mindestens 60% der verbleibenden Mikroorganismen innerhalb von 24 Stunden abgetötet werden.

Diese modifizierten Formkörper haben adhäsionsverringernde Substanzen inkorporiert. Ferner sind deren Oberflächen durch adhäsionsverringernde Substanzen modifiziert.

Außerdem sind biozide Substanzen inkorporiert, wobei als biozide Substanzen Silber, Silberionen freisetzende Substanzen, Kupfer, Kupferionen freisetzende Substanzen, Zink oder Zinkionen freisetzende Substanzen inkorporiert wurden.

Zur Herstellung modifizierter Formkörper werden die adhäsionsverringernden und bioziden Substanzen in die Kunststoffe eingebracht.

Ein Verfahren, z.B. biozide Materialien in die Kunststoffe einbringen, ist die Solvent Casting-Methode. Hierzu werden ausgehärteten Kunststoffe vermahlen, mit Bioziden vermischt und wieder in Form gepresst, ggf. unter Wärmeeinwirkung.

Das Biozid kann auch während des Kunststoff-Spritzvorgangs zugesetzt werden. Bei Abformmassen wird ein Biozid einer beliebigen Komponente der nichtausgehärteten Masse zuzugeben. Durch den Abbindevorgang wird dann ein Gummi erhalten, der biozide Eigenschaften aufweist.

Es kann aber auch während der Polymerisation zugeben werden oder während der Reaktion der Kreuzvernetzung. Schließlich kann Metall (z.B. Ag) mit Zeolith in Partikelform in der Polymerschmelze dispergieren und gemeinsam extrudiert werden.

Alternativ können die adhäsionsverringernden Substanzen auf die Oberfläche der Kunststoffe aufgebracht und die bioziden Substanzen in die Kunststoffe inkorporiert werden.

Diese Kombinationen aus Antihaftfunktion und mikrobiozider Wirkung sind besonders vorteilhaft. So entstehen mikrobiozide Oberflächen von neuer Qualität, die folgende Eigenschaften aufweisen:
∘ Schichten mit niedriger Oberflächenenergie
∘ sehr glatte Oberflächen
∘ Bakterien haften nicht an und wachsen nicht
∘ mikrobiozide Oberflächen mit Schichten, die keimtötende Komponenten enthalten.

Ein Formkörper eines Sauerstoff-Zuschaltventils und/oder Anfeuchters und/oder Beatmungsgerätes und oder Patienteninterfaces und/oder einer Kopfhaube und/oder eines Schlauches und/oder eines Zubehörteiles weist wenigstens abschnittsweise eine über die Oberfläche vermittelte Eigenschaft auf.

## Patentansprüche

1. Beatmungsgerät mit einer Luftfördereinrichtung, an das über einen Atemgasschlauch eine Beatmungsmaske angeschlossen ist, wobei mindestens ein Bereich mindestens eines Bauteiles der Gesamteinrichtung, die aus dem Beatmungsgerät, dem Atemgasschlauch und der Beatmungsmaske besteht, eine Oberfläche aufweist, die keimabweisende und/oder biozide und/oder antimikrobielle Eigenschaften aufweist **dadurch gekennzeichnet, daß** mindestens ein mit einer Oberflächenbeschichtung versehenes Bauteil enthalten ist , wobei dieses Bauteil ein mindestens bereichsweise beschichteter Formkörper aus Kunststoffspritzguß ist und , wobei der Formkörper als Teil eines modulartigen Systems einer Beatmungsmaske ausgebildet ist und zumindest eine Oberflächenbeschichtung des Formkörpers aus dem Material Polysilikon oder Polysiloxan gefertigt ist, wobei der Formkörper mindestens teilweise oder abschnittsweise eine strukturierte Oberfläche sowohl mit regelmäßigen wie mit unregelmäßigen Erhebungen aufweist, wobei die Oberfläche zumindest eine fest verankerte Lage von Mikropartikeln auf, welche Erhebungen bilden und wobei die Erhebungen der Oberfläche des Formkörpers eine Höhe bzw. Tiefe von 5 nm bis 200 *µ*m aufweisen.

2. Verfahren zur Herstellung eines Formkörpers nach Anspruch 1 wobei der Formkörper mittels Kunststoffspritzguß hergestellt und anschließend mindestens bereichsweise beschichtet wird **dadurch gekennzeichnet, daß** wenigstens abschnittsweise die Oberfläche des Formkörpers nach dem Spritzguß mit keimabweisenden Partikeln versehen wird wobei ein Partikel aufweisendes Lösungsmittel durch Aufsprühen, Aufrakeln, Auftropfen oder durch Eintauchen aufgebracht wird.

## Claims

1. Breathing device with an air conveying apparatus to which a breathing mask is connected via a breathing gas hose, wherein at least one area of at least one structural component of the complete apparatus which consists of the breathing device, the breathing gas hose and the breathing mask has a surface which has bacteria-resistant and/or biocidal and/or antimicrobial properties, **characterised in that** at least one structural component provided with a surface coating is contained, wherein this structural component is a moulded part, coated at least in some areas, made by plastic injection moulding and wherein the moulded part is developed as part of a modular system of a breathing mask and at least one surface coating of the moulded part is produced from the material polysilicon or polysiloxane, wherein the moulded part has at least in parts or in sections a structured surface with both regular and irregular elevations, wherein the surface has at least one firmly fixed layer of microparticles which form elevations and wherein the elevations of the surface of the moulded part have a height or depth of 5 nm to 200 µm.

2. Method for the production of a moulded part in accordance with claim 1, wherein the moulded part is produced by plastic injection moulding and then coated at least in some areas, **characterised in that** the surface of the moulded part is provided at least in sections with bacteria-resistant particles after injection moulding, wherein a solvent having particles is applied by spraying, knife-coating, dropwise addition or by immersion.

## Revendications

1. Appareil respiratoire, avec un système de ventilation auquel un masque respiratoire est relié par l'intermédiaire d'un tuyau d'apport de gaz respirable, sachant qu'au moins un secteur d'au moins un élément de l'installation, qui est composée de l'appareil respiratoire, du tuyau d'apport de gaz respirable et du masque respiratoire, est doté d'une surface réfractaires aux germes et / ou qui possède des propriétés biocides et / ou antimicrobiennes,
**caractérisé en ce que** l'un des composants au moins est pourvu d'un revêtement de surface, sachant que ce composant est un corps moulé par injection de matière plastique qui est revêtu, au moins par sections, et sachant que ledit corps moulé est réalisé en tant que composant d'un système modulaire d'un masque respiratoire, et qu'au moins un revêtement de surface du corps moulé est réalisée à partir de polysilicone ou de polysiloxane, sachant que le corps moulé est doté, au moins partiellement ou par sections, d'une surface structurée, qui présente des aspérités aussi bien régulières qu'irrégulières, la surface présentant au moins une couche, fortement ancrée, de microparticules qui forment les aspérités, et sachant que lesdites aspérités de la surface du corps moulé ont une hauteur, respectivement une profondeur de 5 µm à 200 µm.

2. Procédé de fabrication d'un corps moulé selon la revendication 1, sachant que le corps moulé est fabriqué par injection de matière plastique et est revêtu ensuite, au moins par sections,
**caractérisé en ce que** la surface du corps moulé est pourvue, au moins par sections, après le formage par injection, de particules réfractaires aux germes, sachant qu'un solvant, contenant des particules, est appliqué par pulvérisation, par dépôt à la racle, goutte à goutte ou par immersion.
